(19) Europäisches Patentamt
European Patent Office
Office européen des brevets

(11) **EP 3 901 146 A1**

(12) # EUROPEAN PATENT APPLICATION
published in accordance with Art. 153(4) EPC

(43) Date of publication:
**27.10.2021 Bulletin 2021/43**

(21) Application number: **19898039.3**

(22) Date of filing: **17.12.2019**

(51) Int Cl.:
***C07D 401/14*** *(2006.01)* ***C07D 403/14*** *(2006.01)*
***A61K 31/4439*** *(2006.01)* ***A61K 31/496*** *(2006.01)*
***A61K 31/497*** *(2006.01)* ***A61K 31/506*** *(2006.01)*
***A61P 13/10*** *(2006.01)* ***A61P 1/04*** *(2006.01)*
***A61P 11/00*** *(2006.01)* ***A61P 13/00*** *(2006.01)*
***A61P 17/00*** *(2006.01)* ***A61P 19/00*** *(2006.01)*
***A61P 37/00*** *(2006.01)*

(86) International application number:
**PCT/CN2019/125983**

(87) International publication number:
**WO 2020/125627 (25.06.2020 Gazette 2020/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.12.2018 CN 201811553504**
**28.08.2019 CN 201910806179**

(71) Applicant: **Wuhan Humanwell Innovative Drug Research and Development Center Limited Company**
**Wuhan, Hubei 430075 (CN)**

(72) Inventors:
- **ZHANG, Xuejun**
  **Wuhan, Hubei 430075 (CN)**
- **LI, Lie**
  **Wuhan, Hubei 430075 (CN)**
- **SHEN, Jie**
  **Wuhan, Hubei 430075 (CN)**
- **FU, Qiangqiang**
  **Wuhan, Hubei 430075 (CN)**
- **WANG, Yonggang**
  **Wuhan, Hubei 430075 (CN)**

(74) Representative: **Habermann, Hruschka & Schnabel**
**Patentanwälte**
**Montgelasstraße 2**
**81679 München (DE)**

(54) **PHENYLPYRROLIDINE COMPOUND AND USE THEREOF**

(57) Provided is a compound. The compound is a compound represented by formula (A), or a tautomer, a stereoisomer, a hydrate, a solvate, a salt, or a prodrug of the compound represented by formula (A), where R is

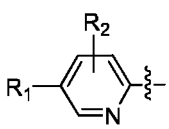 , 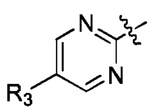 , 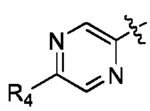 , or 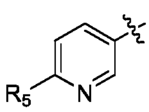 ;

$R_1$ and $R_2$ are each independently selected from H, $C_1$-$C_{10}$ alkyl, $C_3$-$C_{10}$ cycloalkyl, $C_1$-$C_{10}$ alkoxyl, halogen, and -CN; and $R_3$, $R_4$, and $R_5$ are each independently selected from $C_1$-$C_{10}$ alkyl, $C_3$-$C_{10}$ cycloalkyl, $C_1$-$C_{10}$ alkoxyl, halogen, and -CN, under a premise that: if $R_1$ is $-CH_3$, C1, or - CN, $R_2$ is not H, and if $R_1$ is H, $R_2$ is not H, -CN, or $-CH_3$.

EP 3 901 146 A1

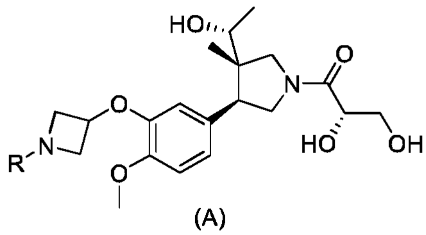

(A)

## Description

### CROSS REFERENCE TO RELATED APPLICATIONS

The present application is based on the following Chinese patent applications and claims priority to these applications, the contents of which are hereby incorporated by reference in their entireties:

- Chinese Application No. 201910806179.2, filed on August 28, 2019; and
- Chinese Application No. 201811553504.0, filed on December 18, 2018.

### FIELD

The present disclosure relates to the fields of chemistry and medicine, and particularly, to a novel phenylpyrrolidine compound and use thereof.

### BACKGROUND

Phosphodiesterase (abbreviated as PDE), also known as 3',5'-cyclic nucleotide phosphodiesterase, is capable of catalyzing the hydrolysis of cyclic adenosine monophosphate (cAMP) to produce 5'-adenosine monophosphate (5'-AMP), or catalyzing the hydrolysis of cyclic guanosine monophosphate (cGMP) to produce 5'-guanosine monophosphate (5'-GMP). Eleven phosphodiesterases have been identified so far. Among them, PDE4, PDE7 and PDE8 have specificity to cAMP. PDE4 is the most important regulator for cAMP expressed in immune and inflammatory cells such as neutrophils, macrophages and T-lymphocytes. cAMP is a crucial second messenger for regulating inflammatory response. It has been reported that PDE4 can also regulate the inflammatory response of inflammatory cells by regulating proinflammatory cytokines such as TNF$\alpha$, IL-2, IFN-$\gamma$, GM-CSF, and LTB4. By inhibiting PDE4, it can effectively treat inflammatory diseases, for example, asthma, chronic obstructive pulmonary disease (COPD), rheumatoid arthritis, atopic dermatitis, inflammatory bowel diseases such as Crohn's disease, and atopic dermatitis (AD).

It is known that PDE4 is divided into four subtypes, i.e., PDE4A, PDE4B, PDE4C, and PDE4D. An inhibition of PDE4D can cause side effects such as vomiting, while a selective inhibition of PDE4B can alleviate the side effects such as vomiting.

In addition, the cyclic nucleotides (cAMP and cGMP) are also important second messengers for regulating the smooth muscle contractility. PDE can hydrolyze the cyclic nucleotides and thus play an important role in regulating the level and duration of action of cellular cyclic nucleotides. Compounds for inhibiting PDE can increase the cellular level of the cyclic nucleotides and relax various types of smooth muscles. Research shows that PDE4 is cAMP-specific and is expressed in the bladder in large quantities. In WO2016040083A1, it is reported that the disclosed azetidinyloxyphenylpyrrolidine compounds can act as PDE4 inhibitors and can be used to treat overactive bladder (OAB), including relief of related symptoms such as frequent urination, urgent urination and other conditions.

Currently, certain progress has been made in the treatment of inflammatory diseases, allergic diseases, and autoimmune diseases. However, it is still required to develop new drugs for improving and replacing the existing drugs.

### SUMMARY

The present disclosure provides a compound capable of effectively inhibiting PDE4, and the compound can be used as an improvement or replacement of the existing drugs or PDE4 inhibitors.

Accordingly, in a first aspect of the present disclosure, the present disclosure provides a compound. The compound is represented by formula (A), or a tautomer, a stereoisomer, a hydrate, a solvate, a salt, or a prodrug of the compound represented by formula (A):

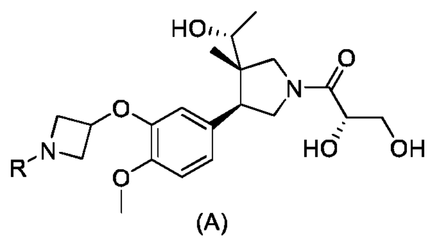

(A)

wherein,

R is

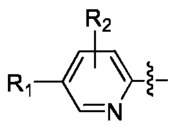, 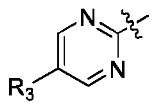, 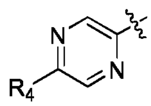 or 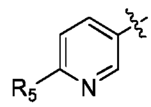;

$R_1$ and $R_2$ are each independently selected from H, $C_1$-$C_{10}$ alkyl, $C_3$-$C_{10}$ cycloalkyl, $C_1$-$C_{10}$ alkoxyl, halogen, and -CN; and

$R_3$, $R_4$ and $R_5$ are each independently selected from $C_1$-$C_{10}$ alkyl, $C_3$-$C_{10}$ cycloalkyl, $C_1$-$C_{10}$ alkoxyl, halogen, and -CN,

under the premise that:

when $R_1$ is $-CH_3$, Cl or -CN, $R_2$ is not H; and

when $R_1$ is H, $R_2$ is not H, -CN or $-CH_3$.

According to an embodiment of the present disclosure, the above compound may also have at least one of the following additional technical features.

According to an embodiment of the present disclosure, $R_1$, $R_3$, $R_4$, and $R_5$ are each independently selected from halogen, $C_1$-$C_5$ alkyl, $C_1$-$C_5$ alkoxyl, halogen, and -CN.

According to an embodiment of the present disclosure, $R_1$, $R_3$, $R_4$, and $R_5$ are each independently selected from halogen, $C_1$-$C_3$ alkyl, $C_1$-$C_3$ alkoxyl, halogen, and -CN.

According to an embodiment of the present disclosure, $R_2$ is selected from halogen, $C_1$-$C_5$ alkyl, $C_1$-$C_5$ alkoxyl, halogen, and -CN.

According to an embodiment of the present disclosure, $R_2$ is selected from halogen, $C_1$-$C_3$ alkyl, $C_1$-$C_3$ alkoxyl, halogen, and -CN.

According to an embodiment of the present disclosure, the halogen is F, Cl, Br, or I.

According to an embodiment of the present disclosure, $R_1$ is selected from $-CH_3$, - $CF_3$, $-CH_2CH_3$, $-CH_2CF_3$, $-OCH_3$, $-OCH_2CH_3$, $-OCH(CH_3)_2$, cyclopropyl, F, Cl, Br, or -CN; $R_2$ is selected from H, -CH3, $-CH_2CH_3$, F, Cl, or -CN; or $R_3$, $R_4$ and $R_5$ are each independently selected from -CH3, $-CF_3$, $-CH_2CH_3$, $-CH_2CF_3$, $-OCH_3$, $-OCH_2CH_3$, $-OCH(CH_3)_2$, cyclopropyl, F, Cl, Br, or -CN.

According to an embodiment of the present disclosure, $R_1$ and $R_2$ are each independently selected from H, $C_1$-$C_6$ alkyl, $C_1$-$C_6$ cycloalkyl, $C_1$-$C_6$ alkoxyl, F, Cl, Br, I, or - CN; and $R_3$, $R_4$ and $R_5$ are each independently selected from $C_1$-$C_6$ alkyl, $C_1$-$C_6$ cycloalkyl, $C_1$-$C_6$ alkoxyl, F, Cl, Br, I, or -CN; under the premise that, when $R_1$ is $-CH_3$, Cl, or -CN, $R_2$ is not H; and when $R_1$ is H, $R_2$ is not H, -CN, or $-CH_3$.

According to an embodiment of the present disclosure, $R_1$ is selected from $-CH_3$, - $OCH_3$, $-OCH_2CH_3$, F, Cl, or -CN; $R_2$ is selected from H, $-CH_3$, F, Cl, or -CN, under the premise that, when $R_1$ is -CH3, Cl, or -CN, $R_2$ is not H; $R_3$ is selected from $C_1$-$C_6$ alkyl, $C_1$-$C_6$ cycloalkyl, F, Cl, Br, or I, preferably methyl, ethyl, or a chlorine atom; $R_4$ is selected from $-CH_3$, $-OCH_3$, F, Cl, or -CN, preferably Cl or -CN; and $R_5$ is selected from $C_1$-$C_6$ alkoxyl, preferably methoxy.

$R_1$ and $R_2$ are each independently selected from H, $C_1$-$C_{10}$ alkyl, $C_3$-$C_{10}$ cycloalkyl, $C_1$-$C_{10}$ alkoxyl, F, Cl, Br, I, -CN; and $R_3$, and $R_4$, and $R_5$ are each independently selected from $C_1$-$C_{10}$ alkyl, $C_3$-$C_{10}$ cycloalkyl, $C_1$-$C_{10}$ alkoxyl, F, Cl, Br, I, or -CN, under the premise that, when $R_1$ is $-CH_3$, Cl, or -CN, $R_2$ is not H; and when $R_1$ is H, $R_2$ is not H, -CN, or $-CH_3$.

According to an embodiment of the present disclosure, $R_1$ is selected from $-CH_3$, - CH2CH3, $-OCH_3$, $-OCH_2CH_3$, $-OCH(CH_3)_2$, F, Cl, or -CN; and $R_2$ is selected from H, $-CH_3$, - $CH_2CH_3$, F, Cl, -CN; under the premise that, when $R_1$ is $-CH_3$, Cl, or -CN, $R_2$ is not a H atom.

According to an embodiment of the present disclosure, $R_3$ is selected from $C_1$-$C_{10}$ alkyl, $C_3$-$C_{10}$ cycloalkyl, F, Cl, Br, or I, preferably methyl, ethyl, or a chlorine atom.

According to an embodiment of the present disclosure, $R_4$ is selected from $-CH_3$, - $OCH_3$, F, Cl, or -CN, preferably Cl or -CN.

According to an embodiment of the present disclosure, $R_5$ is selected from $C_1$-$C_{10}$ alkoxyl, preferably methoxy.

According to an embodiment of the present disclosure, the compound is any one of the following compounds, or the compound is a tautomer, a stereoisomer, a hydrate, a solvate, a pharmaceutically acceptable salt, or a prodrug of any one of the following compounds:

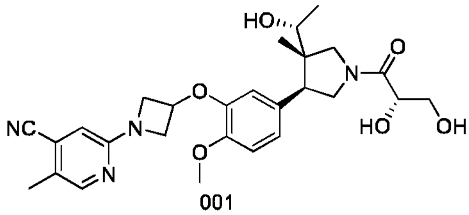

001

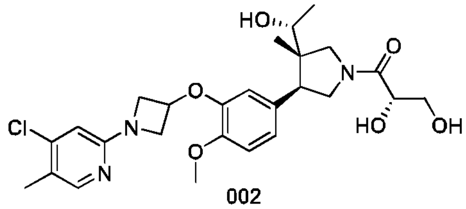

002

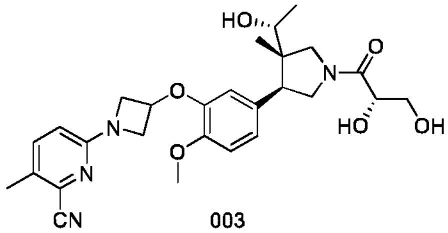

003

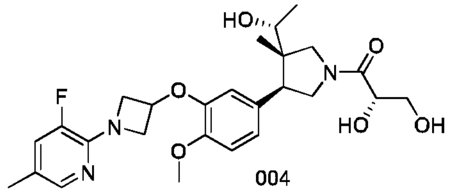

004

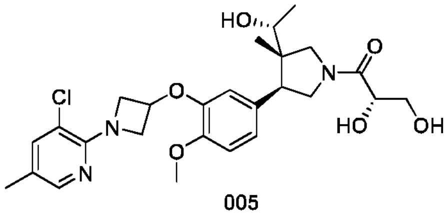

005

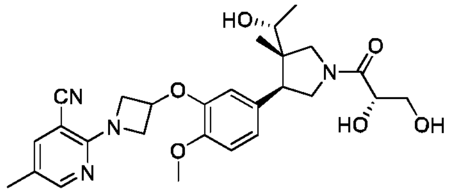

006

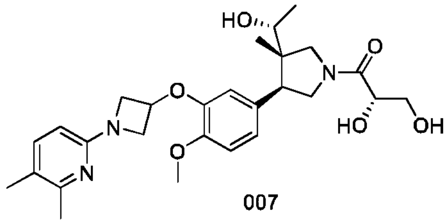

007

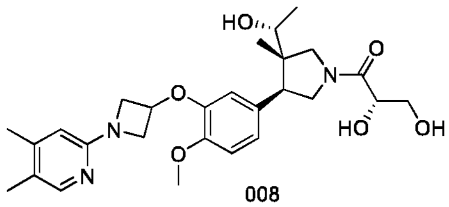

008

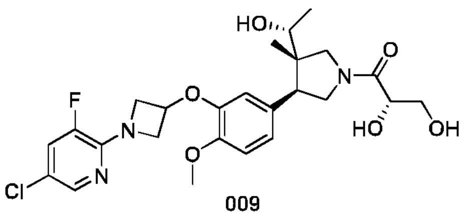

009

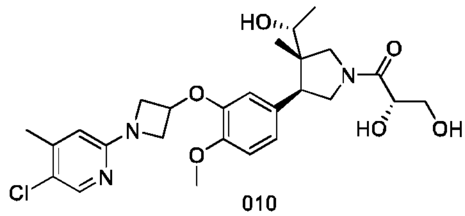

010

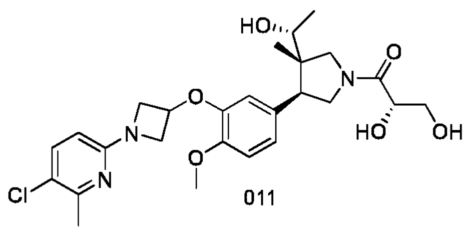

011

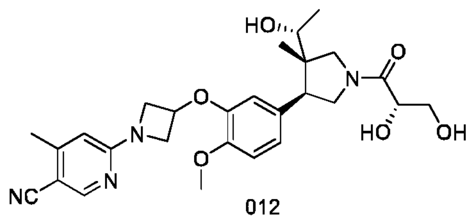

012

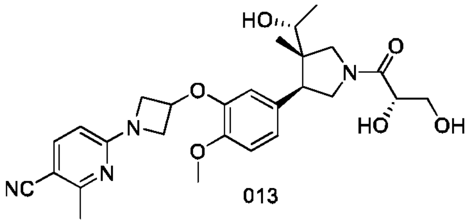

013

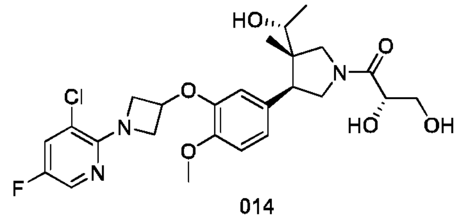

014

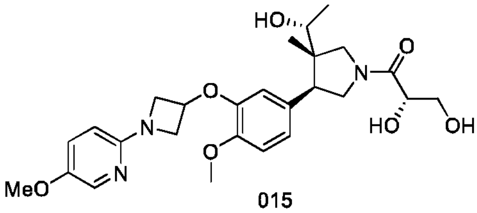

015

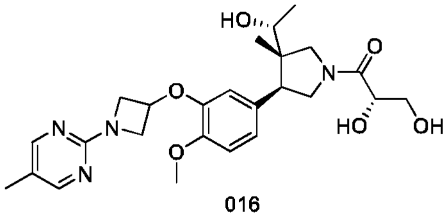

016

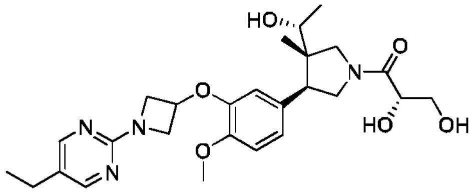

017

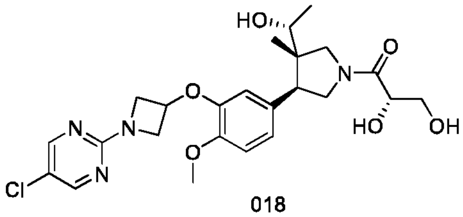

018

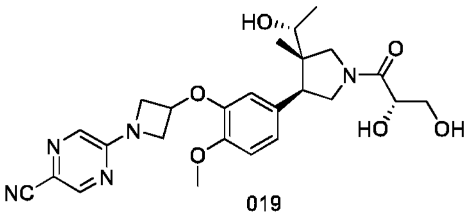

019

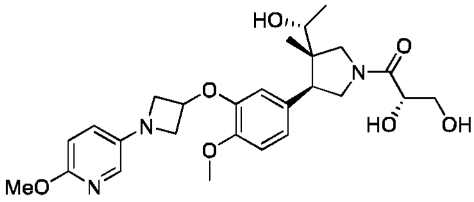

020

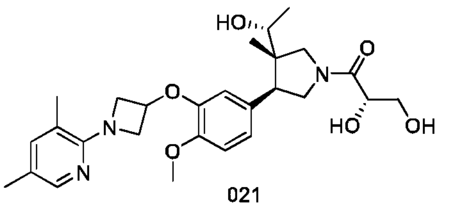

021

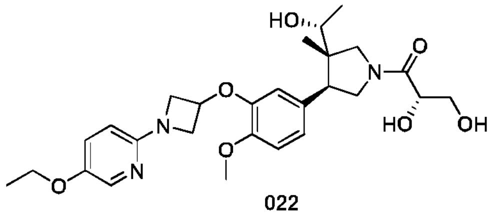

022

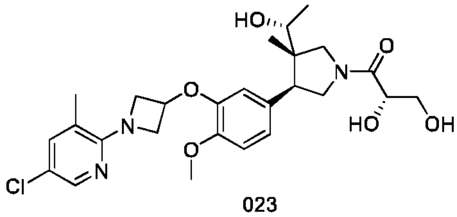

023

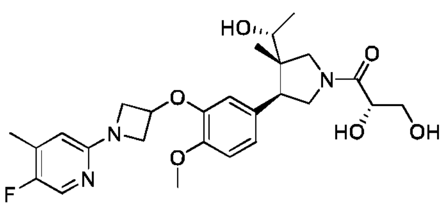

025

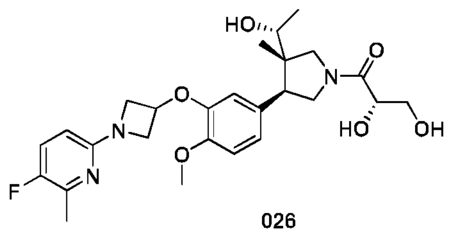

026

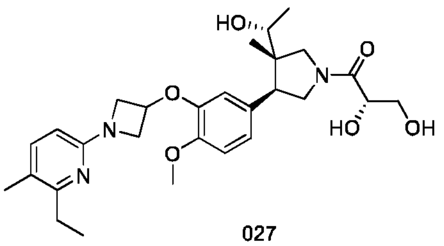

027

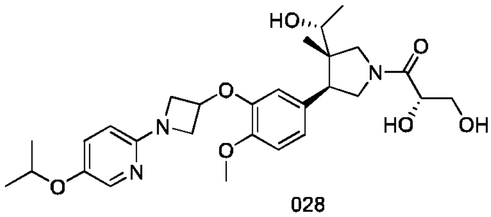

028

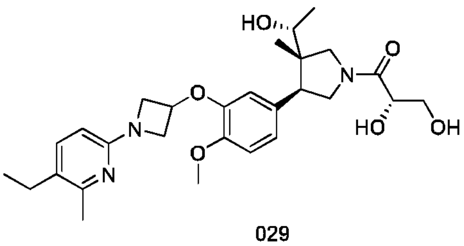

029

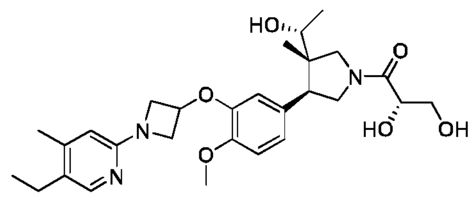

030

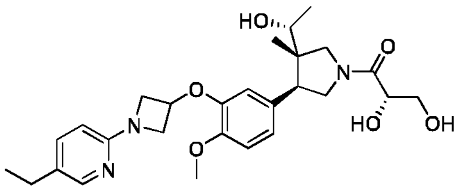

031

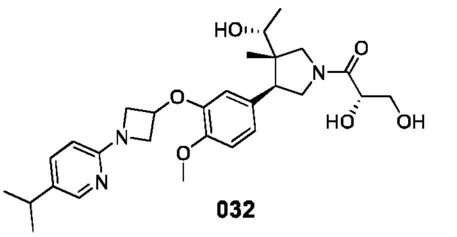

032

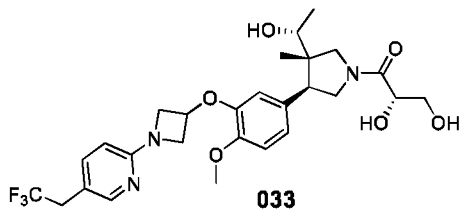

033

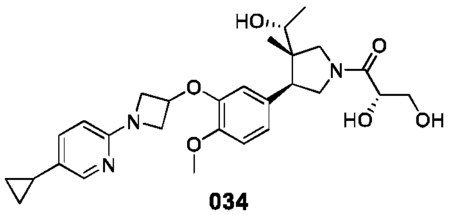

034

According to embodiments of the present disclosure, the salt includes pharmaceutically acceptable salts and is at least one selected from sulfuric acid, phosphoric acid, nitric acid, hydrobromic acid, hydrochloric acid, formic acid, acetic acid, propionic acid, benzenesulfonic acid, benzoic acid, phenylacetic acid, salicylic acid, alginic acid, anthranilic acid, camphoric acid, citric acid, vinyl sulfonic acid, formic acid, fumaric acid, furoic acid, gluconic acid, glucuronic acid,

glutamic acid, glycolic acid, isethionic acid, lactic acid, maleic acid, malic acid, mandelic acid, mucic acid, pamoic acid, pantothenic acid, stearic acid, succinic acid, sulfanilic acid, tartaric acid, *p*-toluenesulfonic acid, malonic acid, 2-hydroxypropionic acid, oxalic acid, glycolic acid, glucuronic acid, galacturonic acid, citric acid, lysine, arginine, aspartic acid, cinnamic acid, *p*-toluenesulfonic acid, methanesulfonic acid, ethanesulfonic acid or trifluoromethanesulfonic acid. Those skilled in the art can understand that, in addition to pharmaceutically acceptable salts, other salts can also be used in the present disclosure, acting as intermediates in the purification of compounds or in the preparation of other pharmaceutically acceptable salts, or for identifying, characterizing or purifying the compounds of the present disclosure.

In a second aspect of the present disclosure, the present disclosure provides a pharmaceutical composition, including the aforementioned compound as an active ingredient; and an additional and selectable drug used for treatment or prevention of at least one of: a PDE4-related disease, an inflammatory disease, an allergic disease, an autoimmune disease, transplant rejection, an arthritic disease, a skin inflammatory disease, an inflammatory bowel disease and a disease related to smooth muscle contractility, asthma, chronic bronchitis, chronic obstructive pneumonia, allergic rhinitis, respiratory distress syndrome, adult respiratory distress syndrome, idiopathic dermatitis, psoriasis, urticaria, rheumatoid arthritis, osteoarthritis, gouty arthritis or spondylitis, ulcerative colitis, Crohn's disease and overactive bladder, frequent urination, and urgent urination. According to an embodiment of the present disclosure, the pharmaceutical composition further includes a pharmaceutically acceptable carrier, a diluent, or an excipient.

In a third aspect of the present disclosure, the present disclosure provides the use of the aforementioned compound or pharmaceutical composition in a preparation of a drug for treatment or prevention of a PDE4-related disease.

In a fourth aspect of the present disclosure, the present disclosure provides the use of the aforementioned compound or pharmaceutical composition in a preparation of a drug for treatment or prevention of at least one disease selected from: an inflammatory disease, an allergic disease, an autoimmune disease, transplant rejection, an arthritic disease, a skin inflammatory disease, an inflammatory bowel disease and a disease related to smooth muscle contractility, asthma, chronic bronchitis, chronic obstructive pneumonia, allergic rhinitis, respiratory distress syndrome, adult respiratory distress syndrome, idiopathic dermatitis, psoriasis, urticaria, rheumatoid arthritis, osteoarthritis, gouty arthritis or spondylitis, ulcerative colitis, Crohn's disease and overactive bladder, frequent urination, and urgent urination.

In a fifth aspect of the present disclosure, the present disclosure provides a drug combination, including the compound described in any one of claims 1 to 9 as an active ingredient, and an additional drug used for treatment or prevention of at least one selected from: a PDE4-related disease, an inflammatory disease, an allergic disease, an autoimmune disease, transplant rejection, an arthritic disease, a skin inflammatory disease, an inflammatory bowel disease and a disease related to smooth muscle contractility, asthma, chronic bronchitis, chronic obstructive pneumonia, allergic rhinitis, respiratory distress syndrome, adult respiratory distress syndrome, idiopathic dermatitis, psoriasis, urticaria, rheumatoid arthritis, osteoarthritis, gouty arthritis or spondylitis, ulcerative colitis, Crohn's disease and overactive bladder, frequent urination, and urgent urination.

According to an embodiment of the present disclosure, by administrating the compound, pharmaceutical composition or drug combination of the present disclosure, patients in need thereof can be provided with better and more effective clinical therapeutic drugs or regimens. According to an embodiment of the present disclosure, the present disclosure provides a series of PDE4 inhibitors with novel structure, strong specificity, excellent efficacy, high bioavailability or good druggability, which can effectively treat PDE4-related diseases or disorders, including, but not limited to, inflammatory diseases, allergic diseases, autoimmune diseases, transplant rejection, arthritic diseases, skin inflammatory diseases, inflammatory bowel disease, and a diseases related to smooth muscle contractility.

Further, the present disclosure relates to the use of the compound represented by general formula (A) or a stereoisomer, a hydrate, a solvate, a pharmaceutically acceptable salt, or a prodrug thereof in preparation of a drug for treating a phosphodiesterase-4 (PDE4) related disease.

In a preferred solution of the present disclosure, the phosphodiesterase-4 (PDE4) related disease is selected from an inflammatory disease, an allergic disease, an autoimmune disease, transplant rejection, an arthritic disease, a skin inflammatory disease, an inflammatory bowel disease, and a disease related to smooth muscle contractility.

In a preferred solution of the present disclosure, the allergic disease is selected from asthma, chronic bronchitis, chronic obstructive pneumonia, allergic rhinitis, or adult respiratory distress syndrome.

In a preferred solution of the present disclosure, the skin inflammatory disease is selected from idiopathic dermatitis, psoriasis, or urticaria.

In a preferred solution of the present disclosure, the arthritic disease is selected from rheumatoid arthritis, osteoarthritis, gouty arthritis, or spondylitis.

In a preferred solution of the present disclosure, the inflammatory bowel disease is selected from ulcerative colitis and Crohn's disease.

In a preferred solution of the present disclosure, the disease related to smooth muscle contractility is selected from overactive bladder and related symptoms, e.g., frequent urination and urgent urination.

The present disclosure also relates to a method for treating phosphodiesterase-4 (PDE4) related diseases, and the method includes, administrating a therapeutically effective dose of a pharmaceutical formulation containing the

compound of the present disclosure or a pharmaceutically acceptable salt thereof to a patient.

Definition and description of terms

Unless otherwise stated, the definitions of groups and terms described in the specification and claims include actual definitions, exemplary definitions, preferred definitions, definitions recorded in tables, and definitions of specific compounds in the examples, etc., which can be arbitrarily combined and integrated with each other. The group definitions and compound structures that are combined and integrated should fall within the scope of the present disclosure.

The term "pharmaceutically acceptable salt" refers to a pharmaceutically acceptable salt of a non-toxic acid or base, including salts of inorganic acids and bases, as well as organic acids and bases. Salts derived from inorganic bases include, but are not limited to, metal salts formed by Al, Ca, Li, Mg, K, Na, and Zn. Salts derived from organic bases include, but are not limited to, salts of primary, secondary or tertiary amines, including organic salts formed by naturally occurring substituted or unsubstituted amines, cyclic amines, and basic ion exchange resins, for example, organic salts formed by ammonium, isopropylamine, trimethylamine, diethylamine, triethylamine, tripropylamine, diethanolamine, ethanolamine, dimethylethanolamine, 2-dimethylaminoethanol, 2-diethylaminoethanol, dicyclohexylamine, caffeine, procaine, choline, betaine, benethamine penicillin, ethylenediamine, glucosamine, methylglucamine, theobromine, triethanolamine, tromethamine, purine, piperazine, piperidine, N-ethylpiperidine, or polyamine resin. Salts derived from inorganic and organic acids include, but are not limited to, organic salts formed by sulfuric acid, phosphoric acid, nitric acid, hydrobromic acid, hydrochloric acid, formic acid, acetic acid, propionic acid, benzenesulfonic acid, benzoic acid, phenylacetic acid, salicylic acid, alginic acid, anthranilic acid, camphoric acid, citric acid, vinyl sulfonic acid, formic acid, fumaric acid, furoic acid, gluconic acid, glucuronic acid, glutamic acid, glycolic acid, isethionic acid, lactic acid, maleic acid, malic acid, mandelic acid, mucic acid, pamoic acid, pantothenic acid, stearic acid, succinic acid, sulfanilic acid, tartaric acid, *p*-toluenesulfonic acid, malonic acid, 2-hydroxypropionic acid, oxalic acid, glycolic acid, glucuronic acid, galacturonic acid, citric acid, lysine, arginine, aspartic acid, cinnamic acid, *p*-toluenesulfonic acid, methanesulfonic acid, ethanesulfonic acid, or trifluoromethanesulfonic acid, etc.

The term "stereoisomer" refers to an isomer produced by a different spatial arrangement of atoms in the molecule, including *cis-/trans*-isomers, enantiomers, diastereomers, and conformational isomers.

The term "tautomer" refers to an isomer of a functional group resulting from a rapid movement of an atom between two positions in a molecule. The compound of the present disclosure may exhibit tautomerism. Tautomeric compounds can be present in two or more mutually convertible species. The prototropy tautomer are resulted from a transfer of covalently bonded hydrogen atoms between two atoms. The tautomer generally exist in an equilibrium form. When trying to separate a single tautomer, a mixture is usually produced, the physical and chemical properties of which are consistent with the mixture of compounds. The position of equilibrium depends on the intramolecularly chemical properties. For example, for many aliphatic aldehydes and ketones, such as acetaldehyde, ketonic type is dominant; and for phenols, enol type is dominant. All tautomeric forms of the compounds are included in the present disclosure.

The term "pharmaceutical composition" refers to a mixture of one or more of the compounds described herein or physiologically/pharmaceutically acceptable salts or prodrugs thereof and other chemical components. The other chemical components can be, for example, physiologically/pharmaceutically acceptable carriers and excipients. The pharmaceutical composition aims to facilitate the administration of the compound to an organism.

The term "solvate" refers to the compound of the present disclosure or a salt thereof including a stoichiometric or non-stoichiometric solvent bonded through an intermolecular noncovalent force. When the solvent is water, the solvate is a hydrate.

The term "prodrug" can be converted into the compound of the present disclosure having biological activity under physiological conditions or through solvolysis. The prodrug of the present disclosure is prepared by modifying the functional groups in the compound, and the modification moiety can be removed by conventional operations or *in vivo,* so as to obtain the parent compound. The prodrug includes a compound, which is formed by connecting a moiety to a hydroxyl group or amino group in the compound of the present disclosure. When the prodrug of the compound of the present disclosure is administered to a mammal individual, the prodrug is dissociated to form a free hydroxyl or amino group.

In the present disclosure, unless otherwise specified, "alkyl" refers to a linear or branched saturated monovalent hydrocarbon group, where one or more hydrogen atoms of the hydrocarbon group may be substituted by halogens such as F, Br, or Cl. As a specific example, hydrogens on one or more carbon atoms can be substituted by halogens such as F, Br, or Cl, and the substituted hydrocarbon group can still be considered as belonging to the category of "alkyl". For example, the term "$C_1$-$C_{10}$ alkyl" can be interpreted to represent a linear or branched saturated monovalent hydrocarbon group having 1, 2, 3, 4, 5, 6, 7, 8, 9, or 10 carbon atoms. The alkyl is, for example, methyl, trifluoromethyl, ethyl, propyl, butyl, pentyl, hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl or

1,2-dimethylbutyl, etc. or isomers thereof. In particular, the group has 1, 2, 3, 4, 5, or 6 carbon atoms ("$C_{1-6}$ alkyl" or "$C_1$-$C_6$ alkyl"), for example, methyl, trifluoromethyl, ethyl, propyl, butyl, isopropyl, isobutyl, sec-butyl, tert-butyl, more particularly, the group has 1, 2 or 3 carbon atoms ("$C_{1-3}$ alkyl" or "$C_1$-$C_3$ alkyl"), for example, methyl, trifluoromethyl, ethyl, n-propyl or isopropyl.

In the present disclosure, unless otherwise specified, "cycloalkyl" should be interpreted to represent a saturated monovalent monocyclic or bicyclic hydrocarbon ring, where one or more hydrogen atoms of the hydrocarbon ring may be substituted by halogens such as F, Br, or Cl. As a specific example, hydrogens on one or more carbon atoms can be substituted by halogens such as F, Br, or Cl. These substituted hydrocarbon rings can still be understood as belonging to the category of "cycloalkyl". For example, the term "$C_3$-$C_{10}$ cycloalkyl" can be interpreted to represent a saturated monovalent monocyclic or bicyclic hydrocarbon ring having 3 to 10 carbon atoms, e.g., cycloheptyl, cyclooctyl, cyclononyl or cyclodecyl, or a bicyclic hydrocarbon group such as a decahydronaphthalene ring. In particular, cycloalkyl may have 3, 4, 5, or 6 carbon atoms ("$C_{3-6}$ cycloalkyl" or "$C_3$-$C_6$ cycloalkyl"), for example, cyclopropyl, cyclobutyl, cyclopentyl, or cyclohexyl.

In the present disclosure, unless otherwise specified, the term "alkoxyl" indicates that a saturated linear or branched alkane is connected to another group via an oxygen atom bond, where one or more hydrogen atoms can be substituted by halogens such as F, Br, or Cl. As a specific example, hydrogens on one or more carbon atoms can be substituted by halogens such as F, Br, or Cl. These substituted groups can still be understood as belonging to the category of "cycloalkyl". Specifically, the term "$C_1$-$C_{10}$ alkoxyl" can indicate that a saturated linear or branched alkyl having 1, 2, 3, 4, 5, 6, 7, 8, 9 or 10 carbon atoms is connected to a structure via an oxygen atom bond, for example, methoxy ($-OCH_3$), ($-OCF_3$)$-O-(CH_2)_9-CH_3$, particularly, the group having 1, 2, 3, 4, 5, or 6 carbon atoms ("$C_{1-6}$ alkoxyl" or "$C_1$-$C_6$ alkoxyl"), for example, $-O-(CH_2)_5-CH_3$.

The term "excipient" refers to a pharmaceutically acceptable inert ingredient. Examples of the "excipient" include, but not limited to, binders, disintegrants, lubricants, glidants, stabilizers, fillers, diluents, and the like. Excipients can improve the properties in term of the processing of the pharmaceutical formulation, i.e., allowing the formulation to be more suitable for direct compression by increasing fluidity and/or adhesion. Examples of typical "pharmaceutically acceptable carriers" suitable for the above formulations are: sugars such as lactose, sucrose, mannitol, and sorbitol; starches such as corn starch, tapioca starch and potato starch; cellulose and its derivatives, such as sodium carboxymethyl cellulose, ethyl cellulose, and methyl cellulose; calcium phosphates, such as dicalcium phosphate, and tricalcium phosphate; sodium sulfate; calcium sulfate; polyvinylpyrrolidone; polyvinyl alcohol; stearic acid; alkaline earth metal salts of stearic acid, such as magnesium stearate and calcium stearate; stearic acid; vegetable oils such as peanut oil, cottonseed oil, sesame oil, olive oil and corn oil; non-ionic, cationic, and anionic surfactants; ethylene glycol polymers; fatty alcohols; and grain hydrolyzed solids and other non-toxic compatible fillers, binders, disintegrants, buffers, preservatives, antioxidants, lubricants, coloring agents, and other excipients commonly used in drug formulations.

The present disclosure also relates to a method for preparing the compound represented by general formula (A) of the present disclosure, and the method includes:

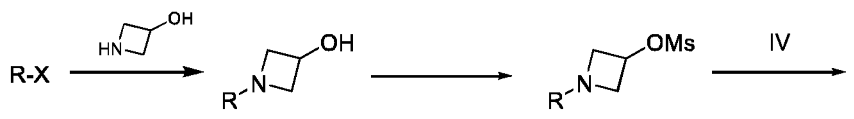


I II III

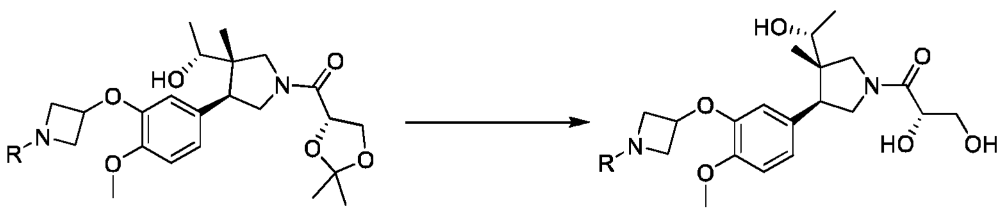

V A

where R is selected from

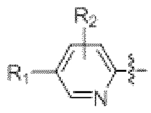, 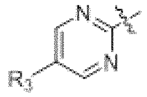, 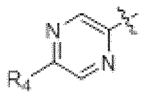, or 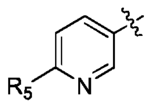;

$R_1$, $R_2$, $R_3$, $R_4$, and $R_5$ are defined as above; X is halogen, such as -Cl, -Br, -I, etc.; and the structure of formula (IV) is

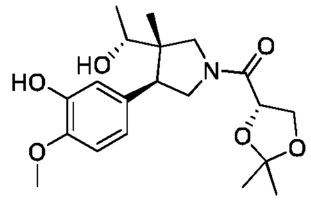.

The compound of formula (I) is subjected to a coupling reaction with 3-azetidinol or a salt thereof under a catalyst to obtain the compound of formula (II); the compound of formula (II) reacts with methylsulfonyl chloride to obtain the compound of formula (III); the compound of formula (III) further undergoes a nucleophilic substitution reaction with the compound of formula (IV) to obtain the compound of formula (V); and the protective moiety of the compound of formula (V) is removed under acidic conditions to obtain the target compound (A).

The synthetic scheme and preparation method of the compound represented by formula (IV) refer to the synthetic methods disclosed in patent applications WO2001047905A1, CN106795137A and the literature by Nichols, P. J.; DeMattei, J. A. Org. Lett. 2006, 8, 1495-1498.

According to the embodiments of the present disclosure, 1) the present disclosure provides PDE4 inhibitors that have novel structures, excellent pharmacokinetic properties, and good efficacy or druggability, suitable for effective treatment of PDE4-related diseases and disorders; and 2) according to the examples of the present disclosure, the compound of the present disclosure has more significant selective inhibitory activity on PDE4B type than the PDE4D type; and compared with the positive control compounds, the compound of the present disclosure has higher PDE4B selectivity and can significantly reduce gastrointestinal side effects such as vomiting caused by PDE4D inhibition.

The additional aspects and advantages of the present disclosure will be partly given in the following description, and part of them will become apparent from the following description, or can be understood through the implementation of the present disclosure.

## BRIEF DESCRIPTION OF DRAWINGS

The foregoing and/or additional aspects and advantages of the present disclosure will become apparent and easy to understand from the description of the embodiments in conjunction with the following drawings, in which:

FIG. 1 illustrates schematic diagrams of clinical scores and inhibition rates based on clinical score AUC obtained in Test Example 5.

## DESCRIPTION OF EMBODIMENTS

The technical solutions of the present disclosure will be described in detail below through embodiments, but the protection scope of the present disclosure is not limited thereto.

Unless otherwise specified, structures of the compounds of the present disclosure are identified by nuclear magnetic resonance (NMR) and/or mass spectrometry (MS). The unit of NMR shift is $10^{-6}$ (ppm). The solvent for NMR measurement is deuterated dimethyl sulfoxide, deuterated chloroform, deuterated methanol, etc., and the internal standard is tetramethylsilane (TMS).

The abbreviations in the present disclosure are defined as follows:

Ms: methylsulfonyl; lipopolysaccharide (LPS): endotoxin; PBMC: peripheral blood monocytes; TNF$\alpha$: tumor necrosis factor $\alpha$; PBS: phosphate buffer solution; FBS: fetal bovine serum; "$IC_{50}$" refers to a half maximal inhibitory concentration, i.e., the concentration at which half of the maximum inhibitory effect can be reached.

Example 1: Preparation of target compound 001 2-(3-(5-((3S,4S)-1-((S)-2,3-dihydroxypropanoyl)-4-((R)-1-hydroxyethyl)-4-methylpyrrolidin-3-yl)-2-methoxyphenoxy)azetidin-1-yl)-5-methylisonicotinonitrile (**target compound 001**)

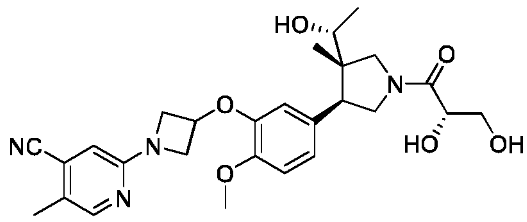

**001**

The synthesis scheme of target compound 001 is as follows:

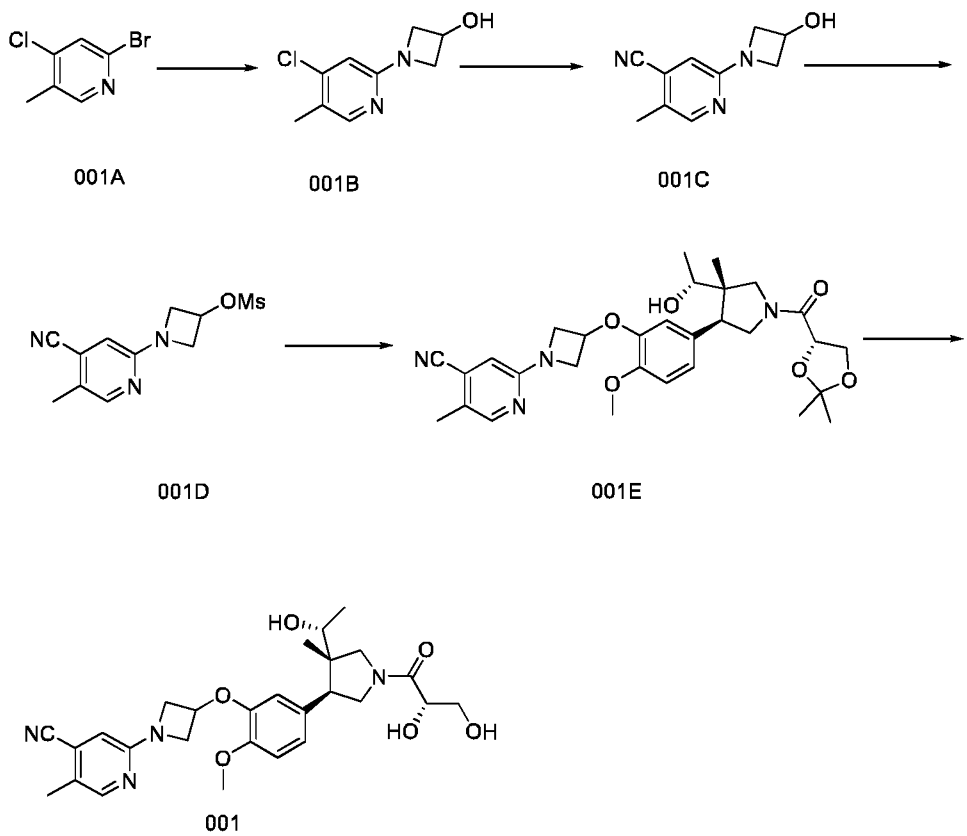

001A 001B 001C

001D 001E

001

Step 1: Synthesis of 1-(4-chloro-5-methylpyridin-2-yl)azetidin-3-ol (**001B**)

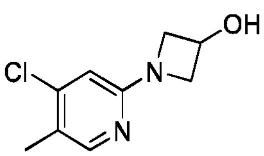

**001B**

At room temperature, 2-bromo-4-chloro-5-methylpyridine (**001A**) (5 g, 25 mmol) was added to 150 ml of DMSO. 3-azetidinol hydrochloride (4.1 g, 37.5 mmol), cuprous iodide (1 g, 5.1 mmol), L-proline (1.15 g, 10 mmol) and potassium carbonate (10.4 g, 75 mmol) were added sequentially. The reaction solution was heated to 90°C and stirred overnight under nitrogen protection. The reaction solution was cooled to room temperature, diluted with distilled water (600 ml), and extracted with ethyl acetate (200 ml×3). The organic phases were combined, washed with saturated brine (200 ml×2), and separated; and the separated organic phase was dried with anhydrous sodium sulfate, filtered, and concentrated. The residue was separated and purified with a silica gel column (petroleum ether: ethyl acetate (V/V) = 2:1) to obtain a solid compound 1-(4-chloro-5)-methylpyridin-2-yl)azetidin-3-ol (**001B**) (4.2 g, yield 84%).
LC-MS, M/Z (ESI): 200 (M+1)

Step 2: Synthesis of 2-(3-hydroxyazetidin-1-yl)-5-methylisonicotinonitrile (**001C**)

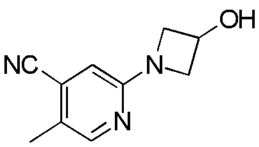

**001C**

1-(4-chloro-5-methylpyridin-2-yl)azetidine-3-ol (**001B**) (30 mg, 0.15 mmol) was dissolved in DMA (4 ml), followed by adding zinc cyanide (53 mg, 0.45 mmol) and tetrakis(triphenylphosphine)palladium (35 mg, 0.03 mmol), microwave heating to 120°C under the protection of nitrogen, and continuing the reaction for 2h. The reaction solution was cooled to room temperature and concentrated under reduced pressure, and the residue was separated and purified by silica gel column (petroleum ether: ethyl acetate (V/V) = 2:1) to obtain the titled compound, 2-(3-hydroxyazetidin-1-yl)-5-methylisonicotinonitrile (**001C**) (10 mg, yield 34.7%).
LC-MS, M/Z (ESI): 190 (M+1)

Step 3: Synthesis of 1-(4-cyano-5-methylpyridin-2-yl)azetidin-3-yl methanesulfonate (**001D**)

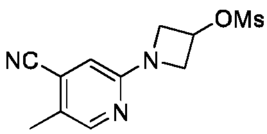

**001D**

2-(3-hydroxyazetidin-1-yl)-5-methylisonicotinonitrile (**001C**) (110 mg, 0.58 mmol) was added to dichloromethane (5 ml), followed by adding triethyl amine (176 mg, 1.7 mmol), cooling to 0°C, adding methylsulfonyl chloride (100 mg, 0.87 mmol), and stirring at room temperature for 2h. The obtained solution was diluted with dichloromethane (20 ml), and the organic phase was washed with deionized water (20 ml×3) and separated. The organic phase was dried with anhydrous sodium sulfate, filtered, and concentrated. The residue was separated and purified with a silica gel column (petroleum ether: ethyl acetate (V/V) = 2:1) to obtain white solid compound 1-(4-cyano-5-methylpyridin-2-yl)azetidin-3-yl methanesulfonate (**001D**) (130 mg, yield 84%).

Step 4: Synthesis of 2-(3-(5-((3S,4S)-1-((S)-2,2-dimethyl-1,3-dioxolane-4-carbo nyl)-4-((R)-1-hydroxyethyl)-4-methylpyrrolidin-3-yl)-2-methoxyphenoxy)azetidin-1-yl)-5-me thylisonicotinonitrile (**001E**)

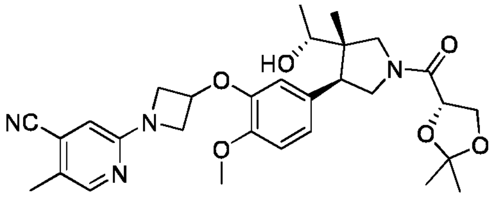

**001E**

Compound ((S)-2,2-dimethyl-1,3-dioxolane-4-yl)((3S,4S)-4-(3-hydroxy-4-methoxyphenyl)-(3-(R)-1-hydroxyethyl)-3-methylpyrrolidin-1-yl))methanone (Compound IV) (150 mg, 0.39 mmol) was added to DMF (2 ml), followed by adding potassium phosphate (339 mg, 1.6 mmol), then heating to 90°C and stirring for 0.5h, cooling to 40°C, adding 1-(4-cyano-5-methylpyridin-2-yl)azetidin-3-yl methanesulfonate (**001D**) (130 mg, 0.48 mmol), heating to 90°C, and stirring overnight. The obtained solution was cooled to room temperature, diluted with distilled water (10 ml), and extracted with ethyl acetate (10 ml×3). The organic phases were combined, washed with saturated brine (10 ml×2), and separated. The separated organic phase was dried with anhydrous sodium sulfate, filtered, and concentrated. The residue was purified with a silica gel column (petroleum ether: ethyl acetate (V/V) = 2:1) to obtain a solid compound 2-(3-(5-((3S,4S)-1-((S)-2,2-dimethyl-1,3-dioxolane-4-carbonyl)-4-((R)-1-hydroxyethyl)-4-methylpyrrolidin-3-yl)-2-methoxyphenoxy)azetidin-1-yl)-5-methylisonicotinonitrile (**001E**) (93 mg, yield 34%).

LC-MS, M/Z (ESI): 551 (M+1).

Step 5: Synthesis of 2-(3-(5-((3S,4S)-1-((S)-2,3-dihydroxypropanoyl)-4-((R)-1-hydroxyethyl)-4-methylpyrrolidin-3-yl)-2-methoxyphenoxy)azetidin-1-yl)-5-methylisonicotinonitrile (**target compound 001**)

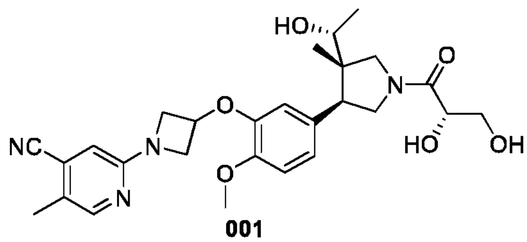

**001**

2-(3-(5-((3S,4S)-1-((S)-2,2-dimethyl-1,3-dioxolane-4-carbonyl)-4-((R)-1-hydroxyethyl)-4-methylpyrrolidin-3-yl)-2-methoxyphenoxy)azetidin-1-yl)-5-methylisonicotinonitrile (**001E**) (82 mg, 0.15 mmol) was dissolved in tetrahydrofuran (4 ml), added with IN hydrochloric acid (2 ml), and stirred at room temperature overnight. pH was adjusted to 8-9 with saturated aqueous sodium bicarbonate solution, and extraction was performed with ethyl acetate (15 ml×3). The organic phases were combined, dried with anhydrous sodium sulfate, filtered, and concentrated. The residue was separated and purify with a silica gel column (pure ethyl acetate), so as to obtain the target compound 2-(3-(5-((3S,4S)-1-((S)-2,3-dihydroxypropanoyl)-4-((R)-1-hydroxyethyl)-4-methylpyrrolidin-3-yl)-2-methoxyphenoxy)azetidin-1-yl)-5-methylisonicotinonitrile (**target compound 001**) (20 mg, yield 26%).

$^1$H NMR (400 MHz, DMSO-d6) δ8.19 (s, 1H), 6.96-6.86 (m, 3H), 6.68 (s, 1H), 5.13 (q, 1H), 4.90 (m, 2H), 4.76 (m, 1H), 4.39 (s, 2H), 4.24 (m, 1H), 3.91 (m, 3H), 3.75 (s, 3H), 3.65-3.34 (m, 6H), 2.29 (s, 3H), 1.02 (d, 3H), 0.64 (s, 3H).

LC-MS, M/Z (ESI): 511 (M+1).

Example 2: Preparation of target compound 002 (S)-1-((3S,4S)-4-(3-((1-(4-chloro-5-methylpyridin-2-yl)azetidin-3-yl)oxy)-4-methoxyphenyl)-3-((R)-1-hydroxyethyl)-3-methylpyrrolidin-1-yl)-2,3-dihydroxypropan-1-one (**target compound 002**))

Using 4-chloro-2-bromo-5-methylpyridine (002A) as a starting material, the **target compound 002** was prepared with the method as described in Step 1 and Step 3 to Step 5 in the preparation method of compound 001. The specific preparation route and method are as follows:

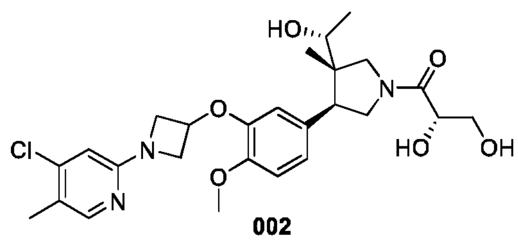

**002**

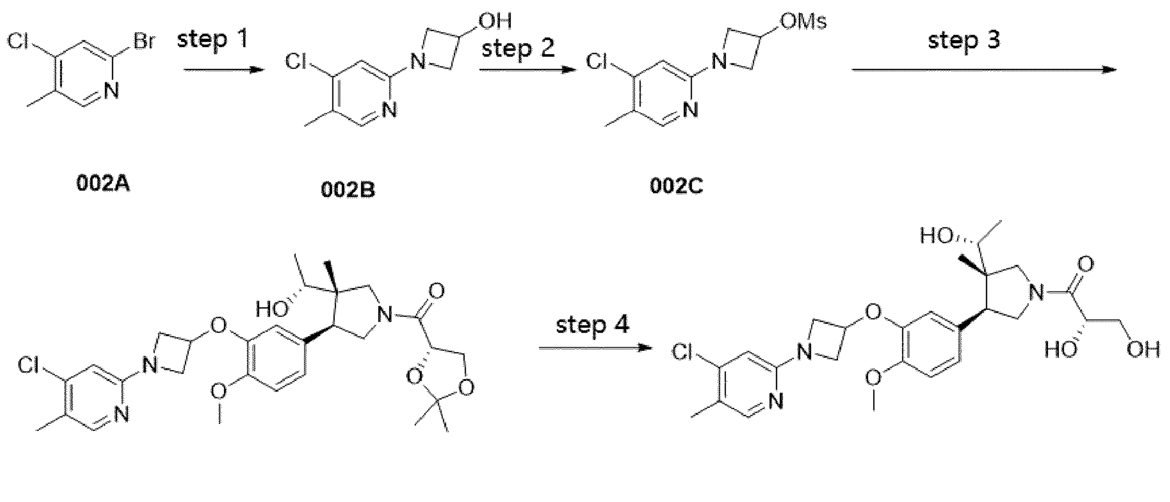


002A 002B 002C

002D 002

Step 1: Synthesis of 1-(4-chloro-5-methylpyridin-2-yl)azetidin-3-ol (**002B**)

4-chloro-2-bromo-5-methylpyridine (**002A**) (5.0 g, 25 mmol) was added to DMSO (150 ml), followed by adding azetidin-3-ol hydrochloride (4.1 g, 37.5 mmol), cuprous iodide (975 mg, 5 mmol), L-proline (1.15 g, 10 mmol) and potassium carbonate (10.4 g, 75 mmol). The reaction solution was heated to 90°C and stirred overnight under the protection of nitrogen. The reaction solution was cooled to room temperature, diluted with deionized water (650 ml), and extracted with ethyl acetate (150 ml × 3). The organic phases were combined, washed with saturated brine (200 ml×2), and separated. The organic phase was dried with anhydrous sodium sulfate, filtered, and concentrated. The residue was separated and purified with a silica gel column (petroleum ether: ethyl acetate (V/V) = 2:1) to obtain the solid compound 1-(4-chloro-5-methylpyridin-2-yl)azetidin-3-ol (**002B**) (4 g, yield 83%).

LC-MS, M/Z (ESI): 199[M+1].

Step 2: Synthesis of 1-(4-chloro-5-methylpyridin-2-yl)azetidin-3-yl methanesulfonate (**002C**)

1-(4-chloro-5-methylpyridin-2-yl)azetidin-3-ol **(002B)** (4 g, 20 mmol) was dissolved in dichloromethane (50 ml), and triethylamine (8.4 ml, 60 mmol) was added. Then, the reaction solution was cooled to 0°C, methylsulfonyl chloride (2.3 ml, 30 mmol) was added dropwise, the temperature was then raised to room temperature, and the reaction solution was stirred for 2h. The obtained solution was diluted with dichloromethane (100 ml), washed with deionized water (60 ml×3), and separated. The separated organic phase was dried with anhydrous sodium sulfate, filtered, and concentrated. The residue was separated and purified with a silica gel column (petroleum ether: ethyl acetate (V/V) = 2:1) to obtain light yellow liquid compound, 1-(4-chloro-5-methylpyridin-2-yl)azetidin-3-yl methanesulfonate (**002C**) (3.5 g, yield 63%).

Step 3: Synthesis of ((3S,4S)-4-(3-((1-(4-chloro-5-methylpyridin-2-yl)azetidin-3-yl)oxy)-4-methoxyphenyl)-3-((R)-1-hydroxyethyl)-3-methylpyrrolidin-1-yl)((S)-2,2-dimethyl-1,3-dioxolan-4-yl)methanone (**002D**)

Compound ((S)-2,2-dimethyl-1,3-dioxolane-4-yl)((3S,4S)4-(3-hydroxy-4-methoxyphenyl)-3-((R)-1-hydroxyethyl)-3-methylpyrrolidin-1-yl)methanone (3.5 g, 9.3 mmol) was dissolved in DMF (70 ml), and potassium phosphate (7.9 g , 37.2 mmol) was added. The reaction solution was heated to 90°C and reacted for 0.5 h. The reaction solution was cooled to 40°C, followed by adding 1-(4-chloro-5-methylpyridin-2-yl)azetidin-3-methylsulfonate (**002C**)

(3.07g, 1.1 mmol), heating to 90°C, and stirring overnight. The obtained solution was cooled to room temperature, diluted with deionized water (400 ml), and extracted with ethyl acetate (100 ml×3). The organic phases were combined, washed with saturated brine (100 ml×2), and separated. The separated organic phase was dried with anhydrous sodium sulfate, filtered, and concentrated. The residue was separated and purified with a silica gel column (petroleum ether: ethyl acetate (V/V) = 2:1) ) to obtain a solid compound, ((3S,4S)-4-(3-((1-(4-chloro-5-methylpyridin-2-yl)azetidin-3-yl)oxy)-4-methoxyphenyl)-3-((R)-1-hydroxyethyl)-3-methylpyrrolidin-1-yl)((S)-2,2-dimethyl-1,3-dioxolan-4-yl)methanone (**002D**) (2.1 g , yield 41%).

LC-MS, M/Z (ESI): 560[M+1].

Step 4: Preparation of (S)-1-((3S,4S)-4-(3-((1-(3-chloro-5-methylpyridin-2-yl)azetidin-3-yl)oxy)-4-methoxyphenyl)-3-((R)-1-hydroxyethyl)-3-methylpyrrolidin-1-yl)-2,3-dihydroxypropan-1-one (**target compound 002**)

((3S,4S)-4-(3-((1-(4-chloro-5-methylpyridin-2-yl)azetidin-3-yl)oxy)-4-methoxyphenyl)-3-((R)-1-hydroxyethyl)-3-methylpyrrolidin-1-yl)((S)-2,2-dimethyl-1,3-dioxolan-4-yl)methanone (**002D**) (2.1 g, 3.75 mmol) was dissolved in tetrahydrofuran (20 ml), followed by adding hydrochloric acid (IN, 10 ml), and stirring at room temperature overnight. The obtained solution was adjusted to pH of 8-9 with a saturated aqueous solution of sodium bicarbonate, and extracted with ethyl acetate (50 ml×3). The organic phases were combined, dried with anhydrous sodium sulfate, filtered, and concentrated. The residue was separated and purified with a silica gel column (pure ethyl acetate) to obtain the target compound, (S)-1-((3S,4S)-4-(3-((1-(3-chloro-5-methylpyridin-2-yl)azetidin-3-yl)oxy)-4-methoxyphenyl)-3-((R)-1-hydroxyethyl)-3-methylpyrrolidin-1-yl)-2,3-dihydroxypropan-1-one (**002**) (1.6 g, 82%).

$^1$H NMR (400 M Hz, DMSO-d6) δ7.99 (s, 1 H), 6.95 (q, 2 H), 6.68 (s, 1 H), 6.59 (s, 1 H), 5.11 (q, 1 H),4.91 (q, 2 H), 4.76 (q, 1 H), 4.36 (q, 2 H), 4.25 (q, 1 H), 3.91 (m, 2 H), 3.75 (s, 3 H), 3.73-3.34 (m, 6 H), 2.16 (s, 3 H), 1.00 (d, 3 H), 0.64 (s, 3 H).

LC-MS, M/Z (ESI): 520 (M+1).

Example 3: Preparation of target compound 003 6-(3-(5-((3S,4S)-1-((S)-2,3-dihydroxypropanoyl)-4-((R)-1-hydroxyethyl)-4-methylpyrrolidin-3-yl)-2-methoxyphenoxy)azetidin-1-yl)-3-methylpicolinonitrile

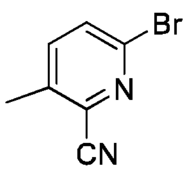

**003A**

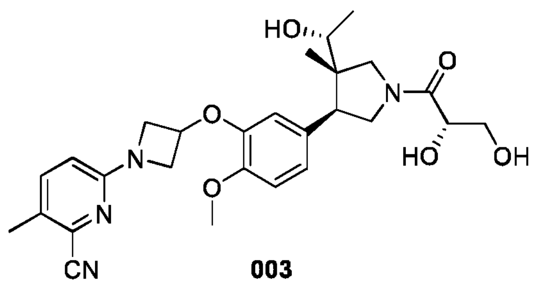

**003**

The **target compound 003** was prepared with the preparation method of compound 002, using 6-bromo-3-methyl-2-cyanopyridine (**003A**) as the starting material.

$^1$H NMR (400 MHz, DMSO-d6) δ 7.64 (q, 1H), 6.96 -6.87 (m, 2H), 6.75 (d, 1H), 6.69 (s, 1H), 5.12 (q, 1H), 4.41 (s, 2H), 4.25 (m, 1H), 3.95 (m, 3H), 3.76 (s, 3H), 3.66-3.48 (m, 7H), 2.33 (s, 3H), 1.02 (d, 3H), 0.65 (s, 3H).

LC-MS, M/Z (ESI): 511 (M+1).

Example 4: Preparation of target compound 004 (S)-1-((3S,4S)-4-(3-((1-(3-fluoro-5-methylpyridin-2-yl)azetidin-3-yl)oxy)-4-methoxyphenyl)-3-((R)-1-hydroxyethyl)-3-methylpyrrolidin-1-yl)-2,3-dihydroxypropan-1-one

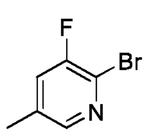

**004A**

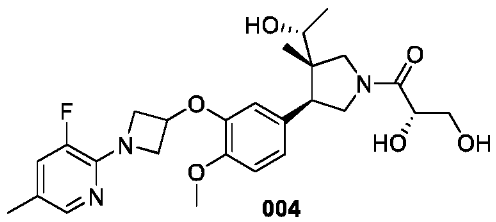

**004**

The **target compound 004** was prepared with the preparation method of compound 002, using 3-fluoro-2-bromo-5-methylpyridine (**004A**) as the starting material.

$^1$H NMR (400 MHz, DMSO-d6) δ7.78 (s, 1 H),7.34 (d, 1 H), 6.94 (d, 1 H), 6.88 (d, 1 H), 6.69 (d, 1 H),5.32 (t,1 H), 5.11 (m, 1 H), 4.86 (m, 2 H), 4.73 (m, 1 H), 4.43 (d, 2 H), 4.24 (d, 2 H),3.95 (m, 2 H), 3.75 (s, 1 H),3.64-3.46 (m, 6 H),2.18 (s, 3 H),1.01 (d, 3 H),0.63 (s, 3 H).

LC-MS, M/Z (ESI): 504 [M+1].

Example 5: Preparation of target compound 005 (S)-1-((3S,4S)-4-(3-((1-(3-chloro-5-methylpyridin-2-yl)azetidin-3-yl)oxy)-4-methoxyphenyl)-3-((R)-1-hydroxyethyl)-3-methylpyrrolidin-1-yl)-2,3-dihydroxypropan-1-one

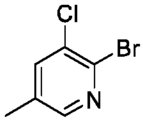
**005A**

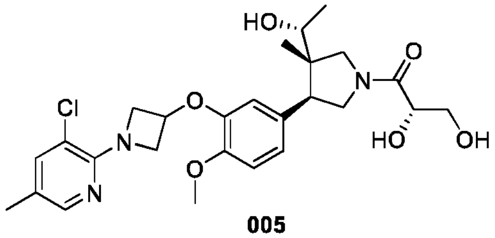
**005**

The **target compound 005** was prepared with the preparation method of compound 002 using 3-chloro-2-bromo-5-methylpyridine (**005A**) as the starting material.

$^{1}H$ NMR (400 MHz, DMSO-d6) δ7.94 (s, 1H), 7.56 (d,1H), 6.93 (q, 1H), 6.88 (d,1 H), 6.69 (d, 1H), 5.07 (q, 1H), 4.52 (q, 4H),4.22 (d, 2H), 4.03 (m, 2H), 3.92 (s, 1H), 3.65 (s, 3H), 3.57-3.47 (m, 6H), 2.17 (s, 3H), 1.01 (d, 3H), 0.63 (s, 3H).

LC-MS, M/Z (ESI): 520[M+1].

Example 6: Preparation of target compound 006 2-(3-(5-((3S,4S)-1-((S)-2,3-dihydroxypropanoyl)-4-((R)-1-hydroxyethyl)-4-methylpyrrolidin-3-yl)-2-methoxyphenoxy)azetidin-1-yl)-5-methylnicotinonitrile

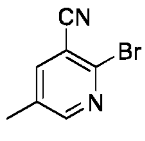
**006A**

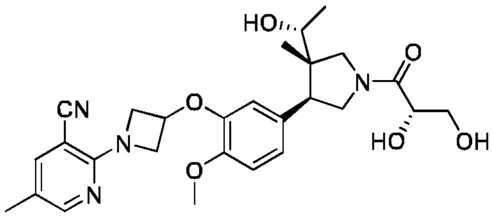
**006**

The **target compound 006** was prepared with the preparation method of compound 002 using 2-bromo-5-methyl-3-cyanopyridine (**006A**) as the starting material.

$^{1}H$ NMR (400 M Hz, DMSO-d6) δ 8.21 (q, 1 H), 7.84 (s, 1 H), 6.97-6.87 (m, 2 H), 6.70 (s, 1 H), 5.12 (q, 1 H), 4.84 (q, 2 H), 4.26 (m,1 H), 4.13 (m,2 H), 3.94 (m, 1 H), 3.74 (s,3 H), 3.67-3.47 (m, 7 H), 2.19 (s, 3 H), 1.01 (d, 3 H), 0.64 (s, 3 H).

LC-MS, M/Z (ESI): 511[M+1].

Example 7: Preparation of target compound 007 (S)-1-((3S,4S)-4-(3-((1-(5, 6-dimethylpyridin-2-yl)azetidin-3-yl)oxy)-4-methoxyphenyl)-3-((R)-1-hydroxyethyl)-3-methylpyrrolidin-1-yl)-2,3-dihydroxypropan-1-one

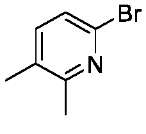
**007A**

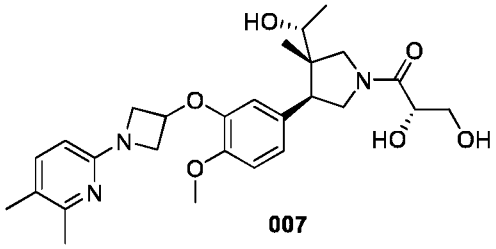
**007**

The **target compound 007** was prepared with the preparation method of compound 002 using 2-bromo-5, 6-dimethylpyridine (**007A**) as the starting material.

$^{1}H$ NMR (400 MHz, DMSO-d6) δ7.29 (s, 1H), 6.94 (m, 2H), 6.68 (s, 1H), 6.23 (m,1H),5.08 (m, 1H), 4.91 (m,

2H), 4.76 (m, 1H), 4.36 (d, 2H), 4.25 (d, 1H), 3.91 (m, 1H), 3.70-3.50 (m, 6H),3.70 (s, 3H), 2.27 (s, 3H), 2.10 (s, 3H), 1.01 (d, 3H), 0.63 (s, 3H).

LC-MS, M/Z (ESI): 500 [M+1].

Example 8: Preparation of target compound 008 (S)-1-((3S,4S)-4-(3-((1-(4,5-dimethylpyridin-2-yl)azetidin-3-yl)oxy)-4-methoxyphenyl)-3-((R)-1-hydroxyethyl)-3-methylpyrrolidin-1-yl)-2,3-dihydroxypropan-1-one

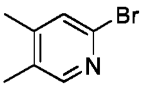

**008A**

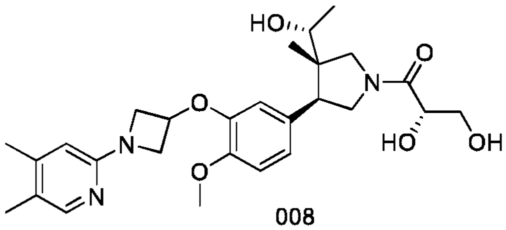

008

The **target compound 008** was prepared with the preparation method of compound 002 using 2-bromo-4,5-dimethylpyridine (**008A**) as the starting material.

$^1$H NMR (400 MHz, DMSO-d6) δ 7.81 (s, 1H), 6.92 (m, 2H), 6.68 (s, 1H), 6.30 (m, 1H), 5.06 (m, 1H), 4.88 (m, 2H), 4.74 (m, 1H), 4.38 (d, 2H), 4.24 (d, 1H), 4.10 (m, 1H), 3.74 (s, 3H), 3.74-3.49 (m, 6H), 2.32 (s, 3H), 2.15 (s, 3H), 1.02 (d, 3H), 0.64 (s, 3H).

LC-MS, M/Z (ESI): 500 [M+1].

Example 9: Preparation of target compound 009 (S)-1-((3S,4S)-4-(3-((1-(5-chloro-3-fluoropyridin-2-yl)azetidin-3-yl)oxy)-4-methoxyphenyl)-3-((R)-1-hydroxyethyl)-3-methylpyrrolidin-1-yl)-2,3-dihydroxypropan-1-one

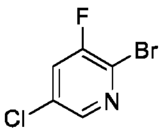

**009A**

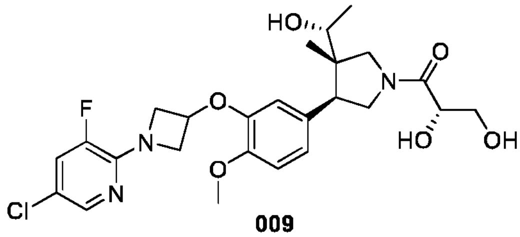

**009**

The **target compound 009** was prepared with the preparation method of compound 002, using 3-fluoro-2-bromo-5-chloropyridine (**009A**) as the starting material.

$^1$H NMR (400 MHz, DMSO-d6) δ8.00 (s, 1H),7.82 (d, 1H), 6.95 (m, 2H), 6.87 (s, 1H),5.14-4.79 (m, 4H),4.52 (d, 2H), 4.26 (d, 1H),4.06 (d, 2H),3.95-3.60 (m, 6H),3.76 (s, 3H), 1.00 (d, 3H), 0.64 (s, 3H).

LC-MS, M/Z (ESI): 524[M+1]

Example 10: Preparation of target compound 010 (S)-1-((3S,4S)-4-(3-((1-(5-chloro-4-methylpyridin-2-yl)azetidin-3-yl)oxy)-4-methoxyphenyl)-3-((R)-1-hydroxyethyl)-3-methylpyrrolidin-1-yl)-2,3-dihydroxypropan-1-one

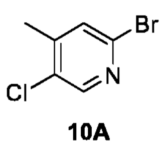

**10A**

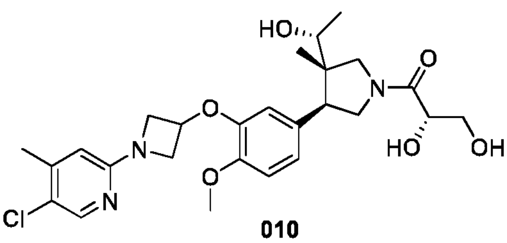

**010**

The **target compound 010** was prepared with the preparation method of compound 002, using 5-chloro-2-bromo-4-methylpyridine (**010A**) as the starting material.

$^1$H NMR (400 MHz, DMSO-d6) δ8.02 (d, 1H), 6.96 (m, 1H), 6.93 (m, 1H), 6.69 (s,1H), 6.48 (s, 1H),5.06 (q,1H), 4.88 (m, 2H), 4.76 (m, 1H), 4.36 (q, 2H),4.24 (m, 1H), 3.91 (m, 2H), 3.76 (s, 3H), 3.66-3.48 (m, 6H), 2.25 (s, 3H), 1.02 (d, 3H), 0.64 (s, 3H)

LC-MS, M/Z (ESI): 520[M+1].

Example 11: Preparation of target compound 011 (S)-1-((3S,4S)-4-(3-((1-(5-chloro-6-methylpyridin-2-yl)azetidin-3-yl)oxy)-4-methoxyphenyl)-3-((R)-1-hydroxyethyl)-3-methylpyrrolidin-1-yl)-2,3-dihydroxypropan-1-one

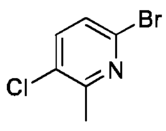

**011A**

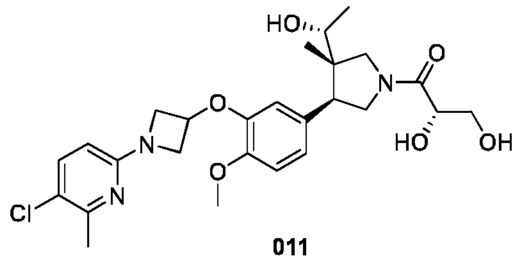

**011**

The **target compound 011** was prepared with the preparation method of compound 002 using 5-chloro-2-bromo-6-methylpyridine (**011A**) as the starting material.

$^1$H NMR (400 MHz, DMSO-d6) δ7.54 (dd, 1H), 6.94 (dd, 1H), 6.87 (d, 1H), 6.68 (s, 1H), 6.33 (d, 1H), 5.10 (m, 1H), 4.88 (m, 2H), 4.74 (dt, 1H), 4.37 (q, 2H), 4.24 (q, 1H), 3.90 (m, 3H), 3.75 (s, 3H), 3.73-3.37 (m, 5H), 2.35 (s, 3H), 1.01 (d, 3H), 0.64 (s, 3H).

LC-MS, M/Z (ESI): 520[M+1].

Example 12: Preparation of target compound 012 6-(3-(5-((3S,4S)-1-((S)-2,3-dihydroxypropanoyl)-4-((R)-1-hydroxyethyl)-4-methylpyrrolidin-3-yl)-2-methoxyphenoxy)azetidin-1-yl)-4-methylnicotinonitrile

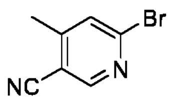

**012A**

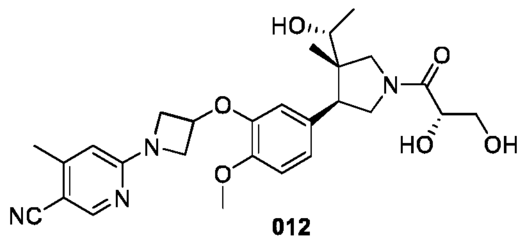

**012**

The **target compound 012** was prepared with the preparation method of compound 002, using 2-bromo-4-methyl-5-cyanopyridine (**012A**) as the starting material.

$^1$H NMR (400 MHz, DMSO-d6) δ8.39 (d, 1H), 6.96 (dd, 1H), 6.89 (d, 1H), 6.69 (s, 1H), 6.45 (s, 1H),5.15 (q, 1H), 4.88 (q, 2H),4.74 (m, 1H), 4.48 (t, 1H), 4.24 (m, 2H), 4.13 (m, 2H), 3.93 (m, 1H), 3.75 (s, 3H), 3.75-3.55 (m, 6H), 2.33 (s, 3H), 1.01 (d, 3H), 0.64 (s, 3H).

LC-MS, M/Z (ESI): 511[M+1].

Example 13: Preparation of target compound 013 6-(3-(5-((3S,4S)-1-((S)-2,3-dihydroxypropanoyl)-4-((R)-1-hydroxyethyl)-4-methylpyrrolidin-3-yl)-2-methoxyphenoxy)azetidin-1-yl)-2-methylnicotinonitrile

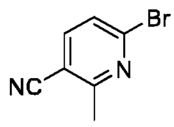

**013A**

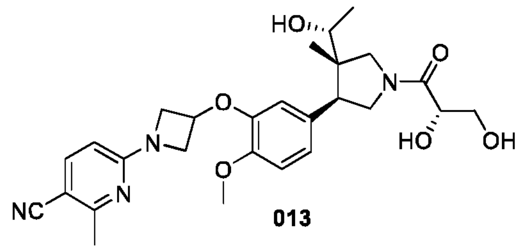

**013**

The **target compound 013** was prepared with the preparation method of compound 002, using 2-bromo-6-methyl-5-cyanopyridine (**7A**) as the starting material.

$^1$H NMR (400 MHz, DMSO-d6) δ7.79 (dd, 1H), 6.94 (m, 1H), 6.90 (m,1H), 6.69 (s, 1H), 6.35 (d, 1H),5.16 (q, 1H), 4.90 (q, 2H), 4.76 (m, 1H), 4.48 (t, 1H), 4.26 (m, 1H), 4.08 (m, 2H), 4.02 (m, 1H), 3.76 (s, 3H), 3.71-3.43 (m, 6H), 2.46 (s, 3H), 1.02 (d, 3H), 0.64 (s, 3H).

LC-MS, M/Z (ESI): 511[M+1].

Example 14: Preparation of target compound 014 (S)-1-((3S,4S)-4-(3-((1-(3-chloro-5-fluoropyridin-2-yl)azetidin-3-yl)oxy)-4-methoxyphenyl)-3-((R)-1-hydroxyethyl)-3-methylpyrrolidin-1-yl)-2, 3-dihydroxypropan-1-one

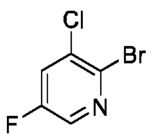

**014A**

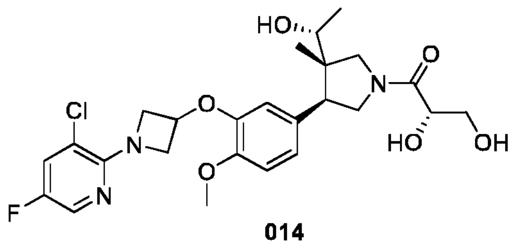

**014**

The **target compound 014** was prepared with the preparation method of compound 002, using 3-chloro-2-bromo-5-fluoropyridine (**014A**) as the starting material.

$^1$H NMR (400 MHz, DMSO-d6) δ8.17 (s, 1H), 7.88 (d, 1H), 6.94 (m, 2H), 6.68 (m, 1H), 5.08 (m, 1H), 4.88 (m, 2H), 4.74 (m, 1H), 4.55 (d, 2H), 4.23 (d, 1H), 4.05 (m, 2H), 3.90-3.67 (m, 6H), 3.75 (s, 3H), 1.01 (d, 3H), 0.62 (s, 3H).

LC-MS, M/Z (ESI): 524 [M+1].

Example 15: Preparation of target compound 015 (S)-2,3-dihydroxy-1-((3S,4S)-3-((R)-1-hydroxyethyl)-4-(4-methoxy-3-((1-(5-methoxypyridin-2-yl)azetidin-3-yl)oxy)phenyl)-3-methylpyrrolidin-1-yl)propan-1-one

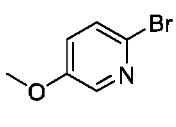

**015A**

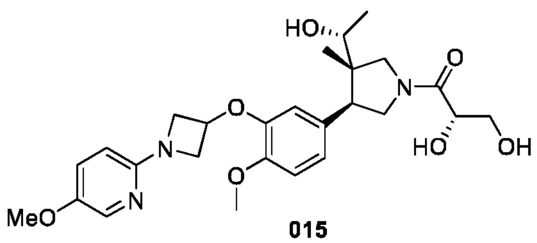

**015**

The **target compound 015** was prepared with the preparation method of compound 002, using 2-bromo-5-methoxypyridine (**015A**) as the starting material.

$^1$H NMR (400 MHz, DMSO-d6) δ7.85 (s, 1H),7.29 (d, 1H), 6.93 (m,2H), 6.69 (s, 1H), 6.45 (d, 1H), 5.09 (m, 1H), 4.88 (m, 2H), 4.72 (m, 1H), 4.31 (m, 3H), 4.25 (d, 1H), 3.92 (d,1H), 3.83 (m, 2H), 3.75 (s, 3H), 3.72-3.45 (m, 6H), 1.02 (d, 3H), 0.64 (s, 3H).

LC-MS, M/Z (ESI): 502 [M+1].

Example 16: Preparation of target compound 016 (S)-2,3-dihydroxy-1-((3S,4S)-3-((R)-1-hydroxyethyl)-4-(4-methoxy-3-((1-(5-methylpyrimidin-2-yl)azetidin-3-yl)oxy)phenyl)-3-methylpyrrolidin-1-yl)propan-1-one

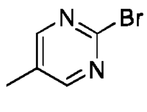

**016A**

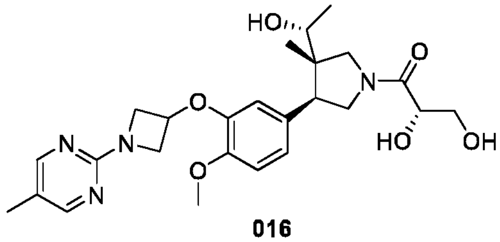

**016**

The **target compound 016** was prepared with the preparation method of compound 002, using 2-bromo-5-methylpyrimidine (**016A**) as the starting material.
$^{1}$H NMR (400 MHz, DMSO-d6) δ8.32 (d, 2H), 6.97 (t, 2H), 6.74 (m, 1H), 5.10 (s, 1H), 4.59 (d, 2H), 4.24 (d, 2H), 4.21 (t, 2H), 3.84 (q, 1H), 3.74-3.30 (m, 6H), 3.74 (s, 3H), 2.20 (s, 3H), 1.14 (d, 3H),0.76 (t, 3H).
LC-MS, M/Z (ESI): 487 [M+1].

Example 17: Preparation of target compound 017 (S)-1-((3S,4S)-4-(3-((1-(5-ethylpyrimidin-2-yl)azetidin-3-yl)oxy)-4-methoxyphenyl)-3-((R)-1-hydroxyethyl)-3-methylpyrrolidin-1-yl)-2,3-dihydroxypropan-1-one

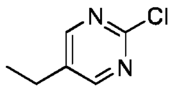

**017A**

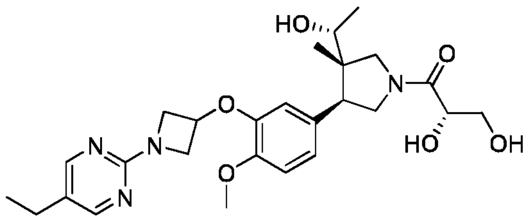

**017**

The **target compound 017** was prepared with the preparation method of compound 002, using 2-chloro-5-ethylpyrimidine (**017A**) as the starting material.
$^{1}$H NMR (400 MHz, DMSO-d6) δ8.37 (d, 2H), 6.97 (t,2H), 6.74 (m, 1H),5.16 (s, 1H), 4.61 (d, 2H), 4.27 (d, 2H), 4.22 (t, 2H), 3.87 (q, 1H), 3.74-3.30 (m, 6H), 3.52 (s, 3H), 2.57 (q, 2H), 1.23 (t, 3H), 1.13 (d, 3H), 0.76 (t, 3H).
LC-MS, M/Z (ESI): 501 [M+1].

Example 18: Preparation of target compound 018 (S)-1-((3S,4S)-4-(3-((1-(5-chloropyrimidin-2-yl)azetidin-3-yl)oxy)-4-methoxyphenyl)-3-((R)-1-hydroxyethyl)-3-methylpyrrolidin-1-yl)-2,3-dihydroxypropan-1-one

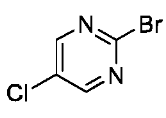

**018A**

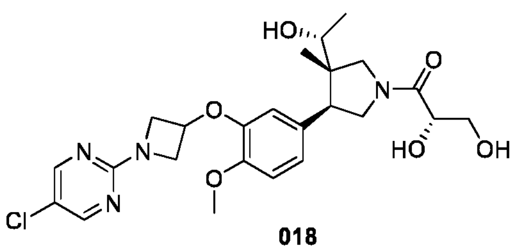

**018**

The **target compound 018** was prepared with the preparation method of compound 002, using 2-bromo-5-chloropyrimidine (**018A**) as the starting material.
$^{1}$H NMR (400 MHz, DMSO-d6) δ8.44 (d, 2H), 6.96 (m,2H), 6.87 (d, 1H), 5.12 (s, 1H), 4.91 (m, 2H), 4.76 (m, 1H), 4.49 (m, 2H), 4.23 (d, 1H), 4.02 (m, 2H), 3.75 (s, 3H), 3.65-3.35 (m, 6H), 1.01 (d, 3H), 0.64 (d, 3H).

LC-MS, M/Z (ESI): 507 [M+1].

Example 19: Preparation of target compound 019 5-(3-(5-((3S,4S)-1-((S)-2,3-dihydroxypropanoyl)-4-((R)-1-hydroxyethyl)-4-methylpyrrolidin-3-yl)-2-methoxyphenoxy)azetidin-1-yl)pyrazine-2-carbonitrile

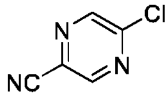

**019A**

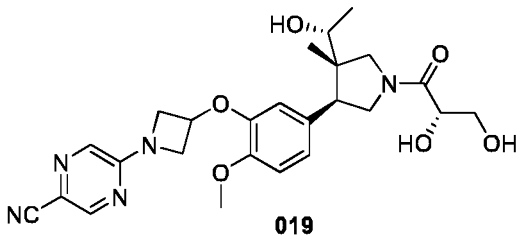

**019**

The **target compound 019** was prepared with the preparation method of compound 002, using 2-cyano-5-chloropiperazine (**019A**) as the starting material.

$^1$H NMR (400 MHz, DMSO-d6) δ8.55 (t, 1H), 8.02 (d, 1H), 6.98 (m, 2H), 6.69 (d, 1H), 5.18 (m, 1H), 4.89 (m, 2H), 4.73 (m, 1H), 4.64 (m, 2H), 4.24 (m, 2H), 3.91 (m, 1H), 3.76 (s, 3H), 3.71-3.36 (m, 6H), 1.02 (d, 3H), 0.65 (d, 3H).

LC-MS, M/Z (ESI): 498 [M+1].

Example 20: Preparation of target compound 020 (S)-2,3-dihydroxy-1-((3S,4S)-3-((R)-1-hydroxyethyl)-4-(4-methoxy-3-((1-(6-methoxypyridin-3-yl)azetidin-3-yl)oxy)phenyl)-3-methylpyrrolidin-1-yl)propan-1-one

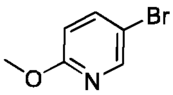

**020A**

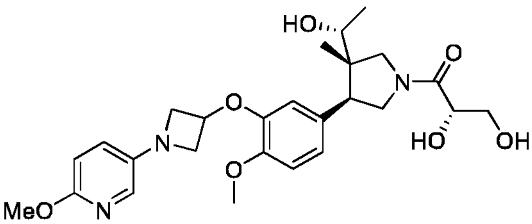

**020**

The **target compound 020** was prepared with the preparation method of compound 002, using 3-bromo-6-methoxypyridine (**020A**) as starting material.

$^1$H NMR (400 MHz, DMSO-d6) δ 7.45 (d, 1H), 7.04 (d, 3H), 6.49 (d, 1H), 6.73 (d, 1H), 5.11 (m, 1H), 4.26 (m, 2H), 3.84 (m, 1H), 3.75-3.35 (m, 16H), 1.03 (d, 3H), 0.64 (d, 3H).

LC-MS, M/Z (ESI): 502 (M+1).

Example 21: Preparation of target compound 021 (S)-1-((3S,4S)-4-(3-((1-(3,5-dimethylpyridin-2-yl)azetidin-3-yl)oxy)-4-methoxyphenyl)-3-((R)-1-hydroxyethyl)-3-methylpyrrolidin-1-yl)-2,3-dihydroxypropan-1-one

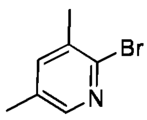

**021A**

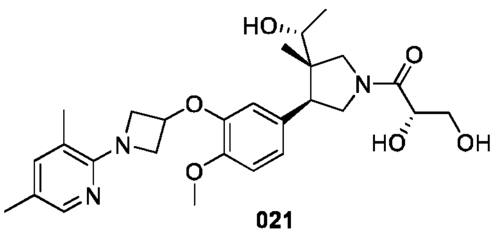

**021**

The **target compound 021** was prepared with the preparation method of compound 002 using 2-bromo-3,5-dimethylpyridine (**021A**) as the starting material.

$^1$H NMR (400 MHz, DMSO-d6) δ 7.80 (s, 1H), 7.19 (s, 1H), 6.93 (dd, 1H), 6.86 (d, 1H), 6.69 (d, 1H), 5.05 (m, 1H), 4.88 (m, 2H), 4.74 (m, 1H), 4.40 (t, 2H), 4.24 (m, 1H), 3.96 (m, 3H), 3.75 (s, 3H), 3.64-3.46 (m, 6H), 2.13 (s, 3H), 2.11 (s, 3H), 1.01 (d, 3H), 0.64 (s, 3H).

LC-MS, M/Z (ESI): 500 (M+1).

Example 22: Preparation of target compound 022 (S)-1-((3S,4S)-4-(3-((1-(5-ethoxypyridin-2-yl)azetidin-3-yl)oxy)-4-methoxyphenyl)-3-((R)-1-hydroxyethyl)-3-methylpyrrolidin-1-yl)-2,3-dihydroxypropan-1-one

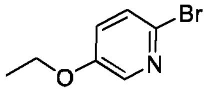

**022A**

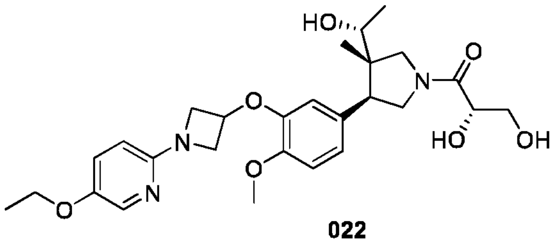

**022**

The **target compound 022** was prepared with the preparation method of compound 002, using 2-bromo-5-ethoxypyridine (**022A**) as the starting material.

$^1$H NMR (400 MHz, DMSO-d6) δ 7.83 (d, 1H), 7.27 (m, 1H), 6.93 (d, 1H), 6.86 (d, 1H), 6.69 (d, 1H), 6.45 (d, 1H), 5.09 (dd, 1H), 4.87 (m, 2H), 4.74 (m, 1H), 4.26 (m, 3H), 3.96 (m, 2H), 3.81 (m, 2H), 3.75 (s, 3H), 3.65-3.51 (m, 6H), 3.17 (m, 1H), 1.29 (t, 3H), 1.03 (d, 3H), 0.64 (d, 3H).

LC-MS, M/Z (ESI): 516 (M+1).

Example 23: Preparation of target compound 023 (S)-1-((3S,4S)-4-(3-((1-(5-chloro-3-methylpyridin-2-yl)azetidin-3-yl)oxy)-4-methoxyphenyl)-3-((R)-1-hydroxyethyl)-3-methylpyrrolidin-1-yl)-2,3-dihydroxypropan-1-one

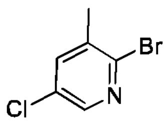

**023A**

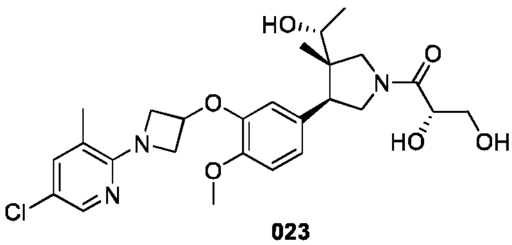

**023**

The **target compound 023** was prepared with the preparation method of compound 002, using 2-bromo-5-chloro-3-methylpyridine **(023A)** as the starting material.

$^1$H NMR (400 MHz, DMSO-d6) δ 7.99 (m, 1H), 7.48 (m, 1H), 6.93 (m, 2H), 6.68 (d, 1H), 5.06 (m, 1H), 4.88 (m, 2H), 4.71 (m, 1H), 4.47 (t, 2H), 4.25 (m, 1H), 4.02 (m, 2H), 3.93 (m, 1H), 3.75 (s, 3H), 3.57-3.48 (m, 7H), 2.16 (s, 3H), 1.02 (d, 3H), 0.64 (s, 3H).

LC-MS, M/Z (ESI): 521 (M+1).

Example 24: Preparation of target compound 024 (S)-1-((3S,4S)-4-(3-((1-(5-chloro-3-fluoropyridin-2-yl)azetidin-3-yl)oxy)-4-methoxyphenyl)-3-((R)-1-hydroxyethyl)-3-methylpyrrolidin-1-yl)-2,3-dihydroxypropan-1-one

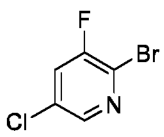

**024A**

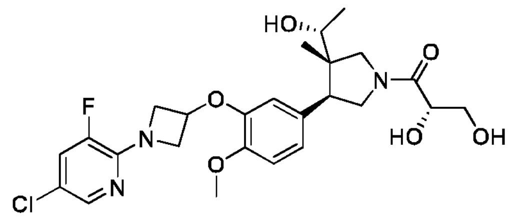

**024**

The **target compound 024** was prepared with the preparation method of compound 002, using 5-chloro-2-bromo-3-fluoropyridine (**024A**) as the starting material.

$^1$H NMR (400 MHz, DMSO-d6) δ 8.00 (s, 1H), 7.78 (dt, 1H), 6.96-6.87 (m, 2H), 6.69 (s, 1H), 5.13-5.12 (m, 1H), 4.52-4.48 (m, 2H), 4.25-4.21 (m, 1H), 4.05-4.00 (m, 2H), 3.93-3.91 (m, 1H), 3.75 (s, 3H), 3.75-3.44 (m, 10H), 1.01 (d, 3H), 0.63 (d, 3H).

LC-MS,M/Z (ESI): 524.2 (M+1).

Example 25: Preparation of target compound 025 (S)-1-((3S,4S)-4-(3-((1-(5-fluoro-4-methylpyridin-2-yl)azetidin-3-yl)oxy)-4-methoxyphenyl)-3-((R)-1-hydroxyethyl)-3-methylpyrrolidin-1-yl)-2,3-dihydroxypropan-1-one

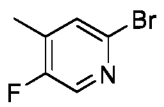

**025A**

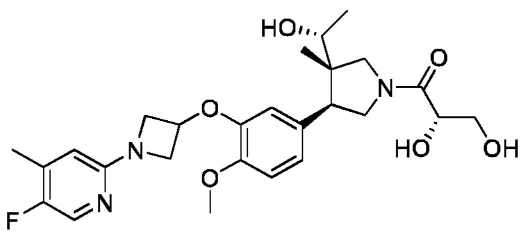

**025**

The **target compound 025** was prepared with the preparation method of compound 002, using 5-fluoro-2-bromo-4-methylpyridine (**025A**) as the starting material.

$^1$H NMR (400 MHz, DMSO-d6) δ8.02 (s, 1H), 6.96-6.87 (m, 2H), 6.68 (s, 1H), 6.53-6.50 (m, 1H), 5.13-5.09 (m, 1H), 4.41-4.37 (m, 4H), 4.25-4.22 (m, 1H), 3.94-3.90 (m, 4H), 3.75 (s, 3H), 3.75-3.47 (m, 6H), 3.35-3.34 (m, 1H), 2.23 (s, 3H), 1.02 (d, 3H), 0.64 (d, 3H).

LC-MS,M/Z (ESI): 504.2 (M+1).

Example 26: Preparation of target compound 026 (S)-1-((3S,4S)-4-(3-((1-(5-fluoro-6-methylpyridin-2-yl)azetidin-3-yl)oxy)-4-methoxyphenyl)-3-((R)-1-hydroxyethyl)-3-methylpyrrolidin-1-yl)-2,3-dihydroxypropan-1-one

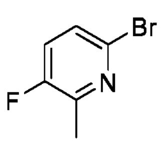

**026A**

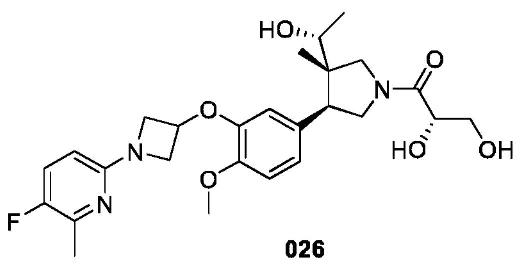

**026**

The **target compound 026** was prepared with the preparation method of compound 002, using 5-fluoro-2-bromo-6-methylpyridine (**026A**) as the starting material.

$^1$H NMR (400 M Hz, DMSO-d6) δ7.45 (t, 1H), 6.95-6.88 (m, 2H), 6.68 (s, 1H), 6.34 (d, 1H), 5.11-5.08 (m, 1H), 4.37-4.33 (m, 2H), 4.27-4.20 (m, 1H), 3.94-3.91 (m, 5H), 3.88 (s, 3H), 3.75-3.40 (m, 6H), 3.36 (dd, 1H), 3.19-3.16 (m, 1H), 2.29 (d, 3H), 1.02 (d, 3H), 0.64 (d, 3H).

LC-MS,M/Z (ESI): 504.2 (M+1).

Example 27: Preparation of target compound 027 (S)-1-((3S,4S)-4-(3-((1-(6-ethyl-5-methylpyridin-2-yl)azetidin-3-yl)oxy)-4-methoxyphenyl)-3-((R)-1-hydroxyethyl)-3-methylpyrrolidin-1-yl)-2,3-dihydroxypropan-1-one

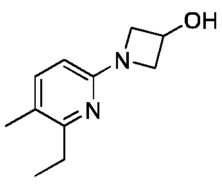

**027D**

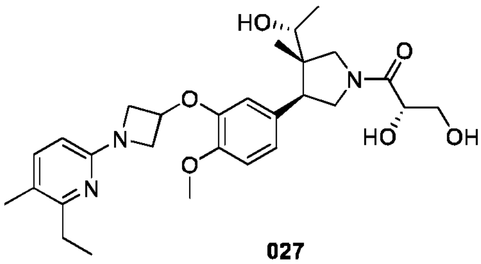

**027**

The **target compound 027** was prepared with the preparation method of compound 002, using 1-(6-ethyl-5-methylpyridin-2-yl)azetidin-3-ol (**027D**) as the starting material.

$^1H$ NMR (400 MHz, $CDCl_3$) $\delta$ 7.56 (d, 1H), 6.87-6.84 (m, 2H), 6.71-6.70 (m, 1H), 6.33 (s, 1H), 5.36-5.34 (m, 1H), 5.08-5.03 (m, 1H), 4.63-4.53 (m, 2H), 4.43-4.38 (m, 2H), 4.23-4.11 (m, 2H), 3.89-3.76 (m, 9H), 3.54-3.50 (m, 1H), 3.43-3.30 (m, 1H), 2.24 (d, 3H), 2.10-2.01 (m, 3H), 1.25-1.14 (m, 6H), 0.76 (d, 3H).

LC-MS, M/Z (ESI): 514.3 (M+1).

1-(6-ethyl-5-methylpyridin-2-yl)azetidin-3-ol **(027D)** was prepared according to the following scheme:

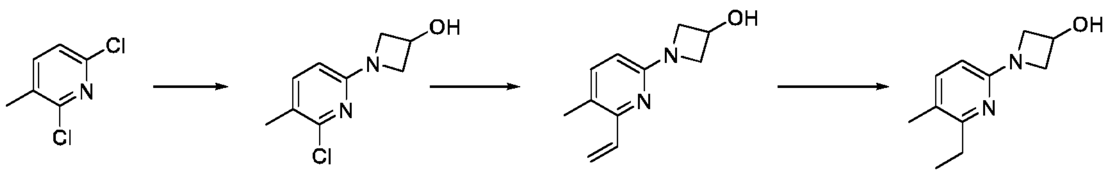

**027A** **027B** **027C** **027D**

Step 1: Synthesis of 1-(6-chloro-5-methylpyridin-2-yl)azetidin-3-ol (**027B**)

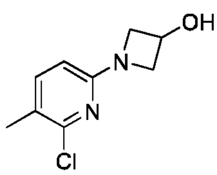

**027B**

2, 6-dichloro-5-methylpyridine **(027A)** (2.0 g, 12.3 mol) was dissolved in DMSO (20 ml), and 3-azetidinol hydrochloride (1.34 g, 12.3 mmol), cuprous iodide (934 mg, 4.92 mmol), L-proline (282 mg, 2.46 mmol), and potassium carbonate (5.09 g, 36.9 mmol) were added. The mixture was heated to 90°C, and stirred overnight under the protection of nitrogen. The obtained solution was cooled to room temperature, diluted with water (100 ml), and extracted with ethyl acetate (30 ml×3). The organic phases were combined, washed with saturated brine (40 ml×2), and separated. The separated organic phase was dried with anhydrous sodium sulfate, filtered, and concentrated. The residue was separated and purified by a silica gel column (petroleum ether: ethyl acetate (V/V) = 2:1) to obtain 1-(6-chloro-5-methylpyridin-2-yl)azetidin-3-ol, white solid (**027B**) (0.4 g, yield 13.8%).

Step 2: Synthesis of 1-(5-methyl-6-vinylpyridin-2-yl)azetidin-3-ol (**027C**)

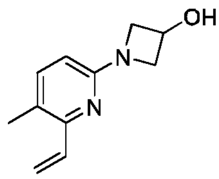

**027C**

1-(6-chloro-5-methylpyridin-2-yl)azetidin-3-ol (**027B**) (310 mg, 1.32 mol) was dissolved in 1,4-dioxane (10 ml), and water (1.0 ml), [1,1'-bis(diphenylphosphino)ferrocene]dichloropalladium(II)-dichloromethane complex (54 mg, 0.066 mmol), potassium vinyltrifluoroborate (530 mg, 3.96 mmol), and potassium carbonate (546 mg, 3.96 mmol) were added. The mixture was micro-wave heated to 120°C under the protection of nitrogen and reacted under stirring for 2 hours. The obtained reaction solution was cooled to room temperature, followed by adding water (50 ml), and extracting with ethyl acetate (20 ml×3). The organic phases were combined, dried with anhydrous sodium sulfate, filtered, and concentrated. The residue was separated and purified with a silica gel column (petroleum ether: ethyl acetate (V/V) = 2:1) to obtain the compound 1-(5-methyl-6-vinylpyridin-2-yl)azetidin-3-ol (027C), white solid (280 mg, 100% yield).

Step 3: Synthesis of 1-(6-ethyl-5-methylpyridin-2-yl)azetidin-3-ol (**027D**)

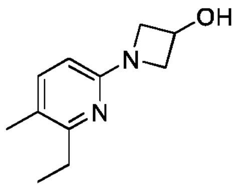

**027D**

The raw material, i.e., 1-(5-methyl-6-vinylpyridin-2-yl)azetidin-3-ol (027C) (0.5 g, 2.63 mmol), was added to ethyl acetate (100 ml), followed by adding 10% Pd/C (0.1 g), introducing hydrogen, and stirring at room temperature for 1 hour. After filtration, the filtrate was concentrated to obtain the compound, 1-(6-ethyl-5-methylpyridin-2-yl)azetidin-3-ol (027D), white solid (0.4g, yield 79.1%).

LC-MS, M/Z (ESI): 193.1 (M+1).

Example 28: Preparation of target compound 028 (S)-2,3-dihydroxy-1-((3S,4S)-3-((R)-1-hydroxyethyl)-4-(3-((1-(5-isopropoxypyridin-2-yl)azetidin-3-yl)oxy)-4-methoxyphenyl)-3-methylpyrrolidin-1-yl)propan-1-one

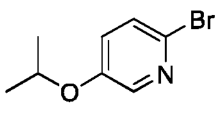

**028A**

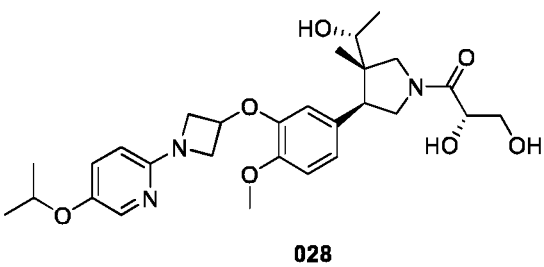

**028**

The **target compound 028** was prepared with the preparation method of compound 002, using 2-bromo-5-isopropoxypyridine (**028A**) as the starting material.

$^{1}H$ NMR (400 MHz, DMSO-d6) δ7.86-7.85 (m, 1H), 7.80-7.77 (m, 1H), 7.70-6.97 (dd, 1H), 6.93-6.88 (m, 2H), 6.72-6.71 (m, 1H), 6.47 (s, 3H), 5.19-5.16 (m, 1H), 4.62-4.59 (m, 2H), 4.30-4.24 (m, 1H), 4.21-4.14 (m, 2H), 3.97-3.94 (m, 2H), 3.77 (s, 3H), 3.74-3.45 (m, 6H), 3.39-3.34 (m, 1H), 1.04 (d, 3H), 0.82-0.78 (m, 2H), 0.71-0.68 (m, 2H), 0.66 (d, 3H).
LC-MS, M/Z(ESI): 530.2 (M+1).

Example 29: Preparation of target compound 029 (S)-1-((3S,4S)-4-(3-((1-(5-ethyl-6-methylpyridin-2-yl)azetidin-3-yl)oxy)-4-methoxyphenyl)-3-((R)-1-hydroxyethyl)-3-methylpyrrolidin-1-yl)-2,3-dihydroxypropan-1-one

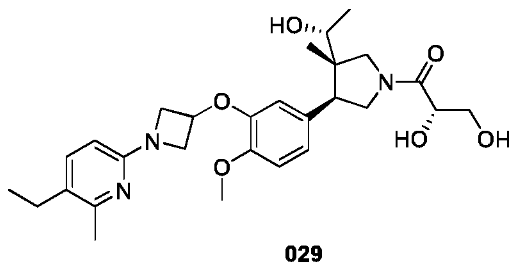

**029**

The specific synthesis scheme and method of compound 029 are as follows:

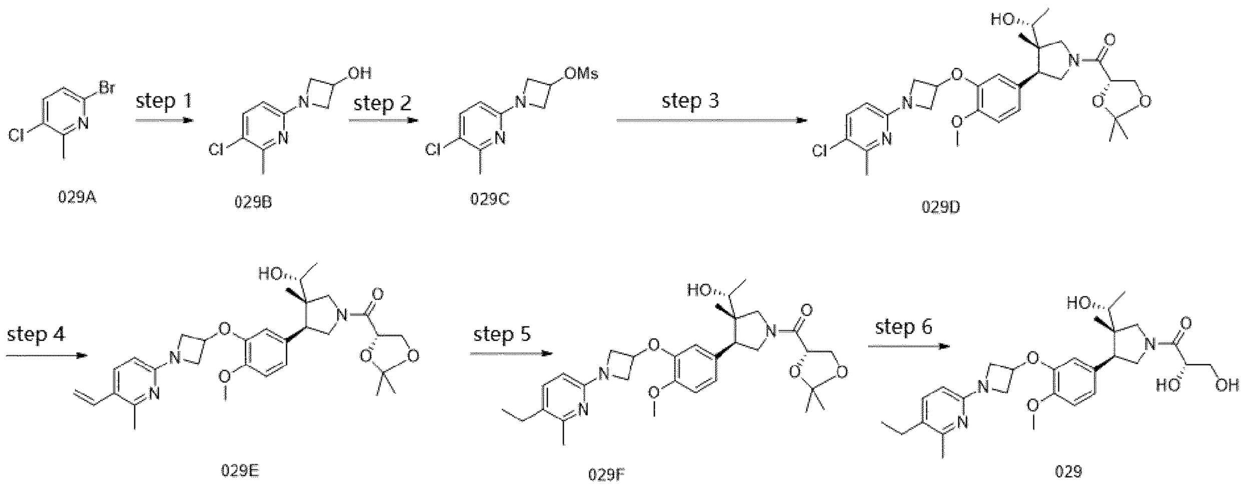


Steps 1 to 3: Synthesis of ((3S,4S)-4-(3-((1-(5-chloro-6-methylpyridin-2-yl)azetidin-3-yl)oxy)-4-methoxyphenyl)-3-((R)-1-hydroxyethyl)-3-methylpyrrolidin-1-yl)((S)-2,2-dimethyl-1,3-dioxolan-4-yl)methanone (**029D**)

The synthesis processes of steps 1 to 3 refer to the foregoing preparation example of compound 002, using 5-chloro-2-bromo-6-methylpyridine (**029A**) as the starting material, to obtain **an intermediate 029D**.

Step 4: Synthesis of ((S)-2,2-dimethyl-1,3-dioxolan-4-yl)((3S,4S)-3-((R)-1-hydroxyethyl)-4-(4-methoxy-3-((1-(6-methyl-5-vinylpyridin-2-yl)azetidin-3-yl)oxy)phenyl)-3-methylpyrrolidin-1-yl)methanone

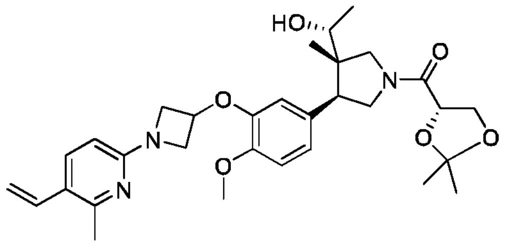

**029E**

((3S,4S)-4-(3-((1-(5-chloro-6-methylpyridin-2-yl)azetidin-3-yl)oxy)-4-methoxyphenyl)-3-((R)-1-hydroxyethyl)-3-methylpyrrolidin-1-yl)((S)-2,2-dimethyl-1,3-dioxolan-4-yl)methanone (**029D**) (70 mg, 0.13 mmol) was dissolved in 1,4-dioxane (3 mL), and water (0.3 ml), chloro(2-dicyclohexylphosphino-2',6'-di-i-propoxy-1,1'-biphenyl) (2-amino-1,1'-biphenyl-2-yl)palladium(II) (5.2 mg, 0.0067 mmol), [1,1'-bis(diphenylphosphino)ferrocene]dichloropalladium(II)-dichloromethane complex (5.5 mg, 0.0067 mmol), and potassium vinyltrifluoroborate (54 mg, 0.403 mmol) were added. The mixture was heated to 140°C and stirred overnight, added with water (10 ml), and extracted with ethyl acetate (10 ml×3). The organic phases were combined, dried with anhydrous sodium sulfate, filtered, and concentrate. The residue was separated and purified with a silica gel column (petroleum ether: ethyl acetate (V/V) = 2:1) to obtain the compound ((S)-2,2-dimethyl-1,3-dioxolan-4-yl)((3S,4S)-3-((R)-1-hydroxyethyl)-4-(4-methoxy-3-((1-(6-methyl-5-vinylpyridin-2-yl)azetidin-3-yl)oxy)phenyl)-3-methylpyrrolidin-1-yl)methanone (**029E**), white solid (6.2 mg, yield 9.0%).

Step 5: Synthesis of ((S)-2,2-dimethyl-1,3-dioxolan-4-yl)((3S,4S)-4-(3-((1-(5-ethyl-6-methylpyridin-2-yl)azetidin-3-yl)oxy)-4-methoxyphenyl)-3-((R)-1-hydroxyethyl)-3-methylpyrrolidin-1-yl)methanone

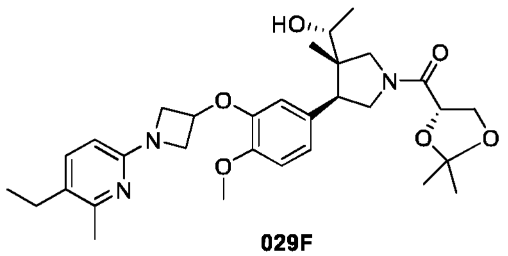

**029F**

((S)-2,2-dimethyl-1,3-dioxolan-4-yl)((3S,4S)-3-((R)-1-hydroxyethyl)-4-(4-methoxy-3-((1-(6-methyl-5-vinylpyridin-2-yl)azetidin-3-yl)oxy)phenyl)-3-methylpyrrolidin-1-yl)methanone (**029E**) (150 mg, 0.27 mmol) was dissolved in 10 ml of methanol, and 10% Pd/C (15 mg) was added, followed by introducing hydrogen gas at room temperature and stirring overnight. The reaction solution was filtered and concentrated to obtain the compound ((S)-2,2-dimethyl-1,3-dioxolan-4-yl)((3S,4S)-4-(3-((1-(5-ethyl-6-methylpyridin-2-yl)azetidin-3-yl)oxy)-4-methoxyphenyl)-3-((R)-1-hydroxyethyl)-3-methylpyrrolidin-1-yl)methanone (029F), white solid (100 mg, yield 66.6%).

Step 6: Synthesis of (S)-1-((3S,4S)-4-(3-((1-(5-ethyl-6-methylpyridin-2-yl)azetidin-3-yl)oxy)-4-methoxyphenyl)-3-((R)-1-hydroxyethyl)-3-methylpyrrolidin-1-yl)-2,3-dihydroxypropan-1-one (**compound 029**)

((S)-2,2-dimethyl-1,3-dioxolan-4-yl)((3S,4S)-4-(3-((1-(5-ethyl-6-methylpyridin-2-yl)azetidin-3-yl)oxy)-4-methoxyphenyl)-3-((R)-1-hydroxyethyl)-3-methylpyrrolidin-1-yl)methanone (**029F**) (100 mg, 0.18 mmol) was dissolved in 10 ml methanol, and hydrochloric acid (IN, 2 ml) was added. The mixture was stirred at room temperature overnight, added with a saturated aqueous solution of sodium bicarbonate to adjust the pH to 8-9, and then extracted with ethyl acetate (25 ml×3). The organic phases were combined, dried with anhydrous sodium sulfate, filtered, and concentrated. The residue was separated and purified with a silica gel column (pure ethyl acetate) to obtain the target compound, (S)-1-((3S,4S)-4-(3-((1-(5-ethyl-6-methylpyridin-2-yl)azetidin-3-yl)oxy)-4-methoxyphenyl)-3-((R)-1-hydroxyethyl)-3-methylpyrrolidin-1-yl)-2,3-dihydroxypropan-1-one (**029**), (45mg, yield 48.5%).

$^1$H NMR (400 MHz, CDCl3) δ 7.29 (t, 1H), 6.85-6.79 (m, 2H), 6.65 (s, 1H), 6.20 (d, 1H), 5.08-5.05 (m, 1H), 4.56-4.54 (m, 1H), 4.43-4.39 (m, 2H), 4.10-4.09 (m, 2H), 3.89-3.76 (m, 8H), 3.56-3.55 (m, 1H), 3.42-3.32 (m, 1H), 2.60-2.48 (m, 2H), 2.40 (s, 3H), 1.26 (t, 3H),1.17 (d, 3H), 0.76 (d, 3H).

LC-MS,M/Z (ESI): 514.2 (M+1).

Example 30: Preparation of target compound 030 (S)-1-((3S,4S)-4-(3-((1-(5-ethyl-4-methylpyridin-2-yl)azetidin-3-yl)oxy)-4-methoxyphenyl)-3-((R)-1-hydroxyethyl)-3-methylpyrrolidin-1-yl)-2,3-dihydroxypropan-1-one

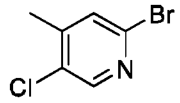

**030A**

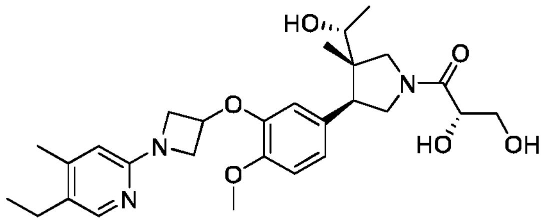

**030**

The **target compound 030** was prepared with the preparation method of compound 029, using 5-chloro-2-bromo-4-methylpyridine (**030A**) as the starting material.

$^{1}H$ NMR (400 MHz, $CDCl_3$) δ 7.28 (m, 1H), 6.85-6.79 (m, 2H), 6.65 (s, 1H), 6.20 (d, 1H), 5.08-5.05 (m, 1H), 4.58-4.53 (m, 1H), 4.43-4.39 (m, 2H), 4.16-4.08 (m, 2H), 3.89-3.76 (m, 9H), 3.58-3.53 (m, 1H), 3.43-3.32 (m, 1H), 2.60-2.48 (m, 5H), 2.40 (d, 3H), 1.26-1.13 (m, 6H), 0.76 (d, 3H).

LC-MS,M/Z (ESI): 514.3 (M+1).

Example 31: Preparation of target compound 031 (S)-1-((3S,4S)-4-(3-((1-(5-ethylpyridin-2-yl)azetidin-3-yl)oxy)-4-methoxyphenyl)-3-((R)-1-hydroxyethyl)-3-methylpyrrolidin-1-yl)-2,3-dihydroxypropan-1-one

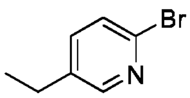

**031A**

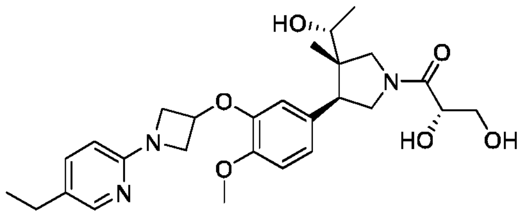

**031**

The **target compound 031** was prepared with the preparation method of compound 002, using 2-bromo-5-ethylpyridine (**031A**) as the starting material.

$^{1}H$ NMR (400 MHz, DMSO-d6) δ7.94 (s, 1H), 7.42 (dt, 1H), 6.94 (dd, 1H), 6.87 (d, 1H), 6.69 (d, 1H), 6.42 (d, 1H), 5.12-5.09 (m, 1H), 4.92-4.87 (m, 2H), 4.74 (m, 1H), 4.33 (m, 3H), 4.24 (m, 1H), 3.27-3.90 (m, 1H), 3.86-3.83 (m, 2H), 3.75 (s, 3H), 3.70-3.45 (m, 6H), 3.37-3.32 (m, 1H), 2.50-2.44 (m, 1H), 1.12 (t, 3H), 1.01 (d, 3H), 0.64 (d, 3H).

LC-MS, M/Z (ESI):500.3 (M+1).

Example 32: Positive control compound and its preparation

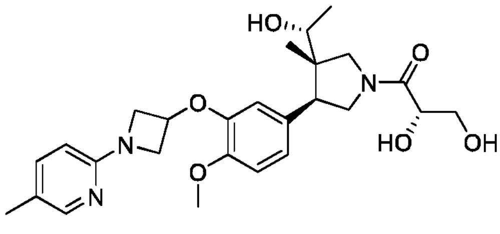

**Positive control compound**

The positive control compound was prepared with the preparation method referring to the patent WO2014159012A1.

LC-MS, M/Z (ESI): 486 [M+1].

The positive control compound mentioned in the following test examples all refers to the compound in Example 32.

Example 33: Preparation of target compound 032 (S)-2,3-dihydroxy-1-((3S,4S)-3-((R)-1-hydroxyethyl)-4-(3-((1-(5-isopropylpyridin-2-yl)azetidin-3-yl)oxy)-4-methoxyphenyl)-3-methylpyrrolidin-1-yl)propan-1-one (**compound 032**)

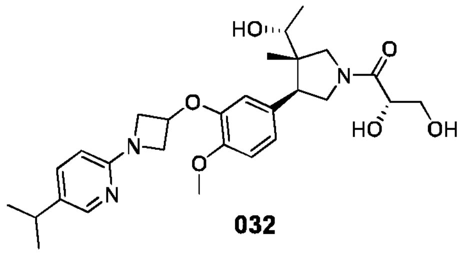

**032**

The specific synthesis scheme and method of compound 032 are as follows:

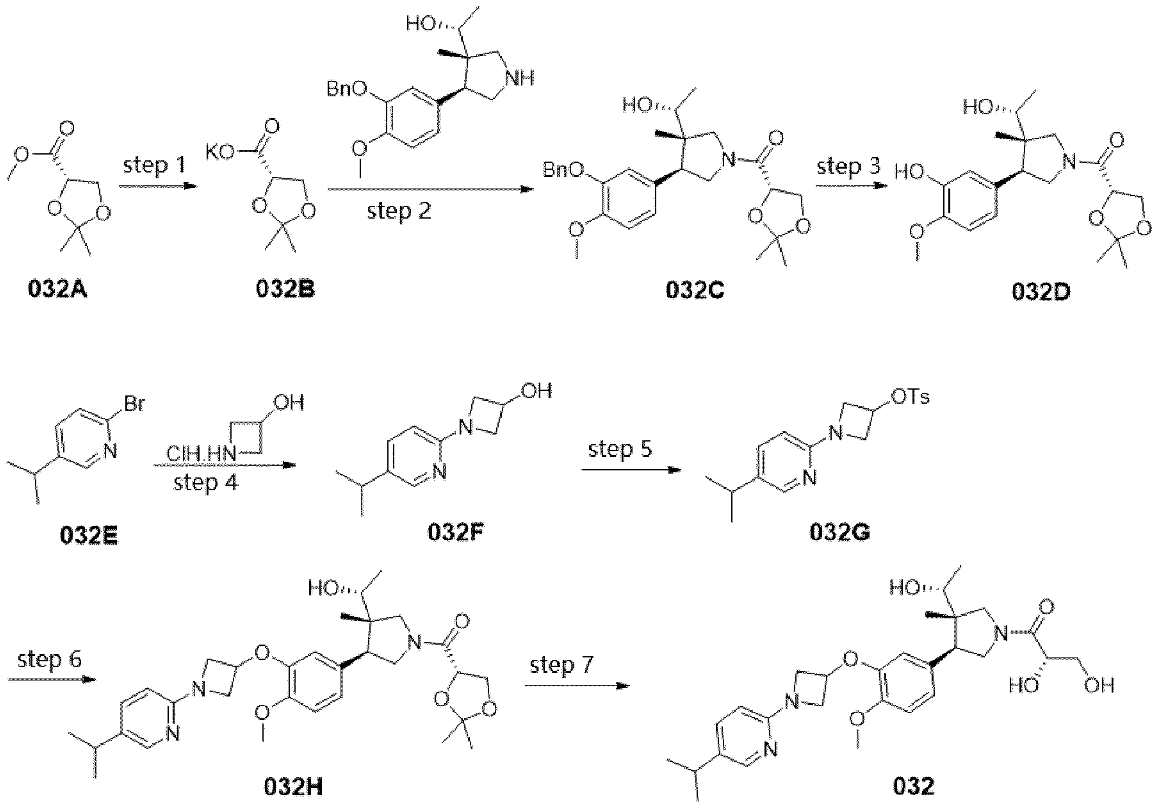


032A 032B 032C 032D 032E 032F 032G 032H 032

Step 1: Synthesis of potassium (S)-2,2-dimethyl-1,3-dioxolane-4-carboxylate (**032B**)

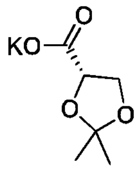

**032B**

At room temperature, methyl (S)-2,2-dimethyl-1,3-dioxolane-4-carboxylate (**032A**) (10 g, 62.4 mmol) was added to 200 ml of methanol, and the reaction solution was heated to 50°C and reacted under stirring for 2h. The completion of the reaction was monitored by TLC (PE: EA=10:1) with potassium permanganate as the chromogenic agent. The reaction solution was concentrated and dried to obtain light yellow solid as crude product of **compound 032B** (11.6 g, yield 101%), which could be directly put into the next step of reaction.

Step 2: Synthesis of ((3S,4S)-4-(3-(benzyloxy)-4-methoxyphenyl)-3-((R)-1-hydroxyethyl)-3-methylpyrrolidin-1-yl)((S)-2,2-dimethyl-1,3-dioxolan-4-yl)methanone **(032C)**

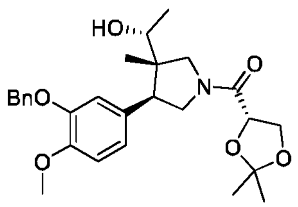

**032C**

At room temperature, potassium (S)-2,2-dimethyl-1,3-dioxolane-4-carboxylate (**032B**) (11.5 g, 62.4 mmol) was added to 150 mL of DMF solution, then (R)-1-((3S,4S)-4-(3-(benzyloxy)-4-methoxyphenyl)-3-methylpyrrolidin-3-yl)ethan-1-ol (7.1 g, 20.8 mmol) was added, the reaction solution was cooled to 0°C, and 50% 1-propylphosphonic anhydride in DMF solution (19.8 g, 31.2 mmol) was added dropwise. Then, the mixture was slowly warmed to room temperature, and stirred under the protection of nitrogen overnight. The solution was diluted with saturated sodium chloride solution (100 mL) and extracted with ethyl acetate (100 mL×3). The organic phases were combined, washed with saturated brine (100 mL×2), and separated. The separated organic phase was dried with anhydrous sodium sulfate, filtered, and concentrated. The residue was separated and purified with a silica gel column (petroleum ether: ethyl acetate (V/V) = 2:1) to obtain a colorless oily compound ((3S,4S)-4-(3-(benzyloxy)-4-methoxyphenyl)-3-((R)-1-hydroxyethyl)-3-methylpyrrolidin-1-yl)((S)-2,2-dimethyl-1,3-dioxolan-4-yl)methanone (**032C**) (6.1 g, yield 62.5%).

LC-MS, M/Z (ESI): 470.58 (M+1)

Step 3: Synthesis of (S)-2,2-dimethyl-1,3-dioxolan-4-yl)((3S,4S)-4-(3-hydroxy-4-methoxyphenyl)-3 -((R)-1 -hydroxyethyl)-3 -methylpyrrolidin-1 -yl)methanone (**032D**)

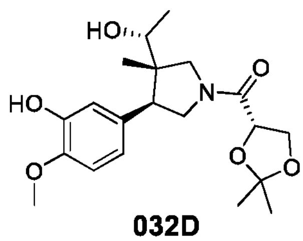

**032D**

At room temperature, ((3S,4S)-4-(3-(benzyloxy)-4-methoxyphenyl)-3-((R)-1-hydroxyethyl)-3-methylpyrrolidin-1-yl)((S)-2,2-dimethyl-1,3-dioxolan-4-yl)methanone (**032C**) (6.1 g, 8.12 mmol) was added to 100 mL of methanol solution, stirred overnight at room temperature in a hydrogen atmosphere, and filtered through celite. The filter cake was washed

with methanol (50 mL×3), and the filtrate was concentrated to dryness to obtain a colorless oily compound (S)-2,2-dimethyl-1,3-dioxolan-4-yl)((3S,4S)-4-(3-hydroxy-4-methoxyphenyl)-3-((R)-1-hydroxyethyl)-3-methylpyrrolidin-1-yl)methanone (**032D**) (3.8 g, yield 77%).

LC-MS, M/Z (ESI): 380.45 (M+1)

Step 4: Synthesis of 1-(5-isopropylpyridin-2-yl)azetidin-3-ol (**032F**)

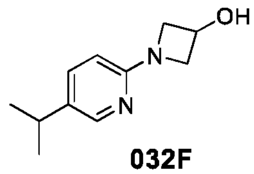

**032F**

At room temperature, 2-bromo-5-isopropylpyridine (032E) (2 g, 10 mmol) was added to 10 mL of DMSO, and 3-azetidinol hydrochloride (1.5 g, 14.99 mmol), cuprous iodide (1.9 g, 10.0 mmol), L-proline (0.23 g, 2.0 mmol) and cesium carbonate (6.51 g, 20 mmol) were added sequentially. The reaction solution was heated to 95°C and stirred overnight under the protection of nitrogen. The reaction solution was cooled to room temperature, diluted with water (20 mL), and extracted with ethyl acetate (100 mL × 3). The organic phases were combined, washed with saturated brine (100 mL×2), and separated. The separated organic phase was dried with anhydrous sodium sulfate, filtered, and concentrated. The residue was separated and purified by a silica gel column (petroleum ether: ethyl acetate (V/V) = 2:1) to obtain white solid compound, 1-(5-isopropylpyridin-2-yl)azetidin-3-ol (**032F**) (1.0 g, yield 52%).

LC-MS, M/Z (ESI): 193.26 (M+1)

Step 5: Synthesis of 1-(5-isopropylpyridin-2-yl)azetidin-3-yl 4-methylbenzenesulfonate **(032G)**

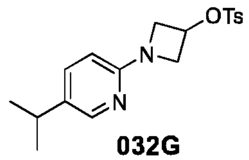

**032G**

1-(5-isopropylpyridin-2-yl)azetidin-3-ol (**032F**) (1.0 g, 5.2 mmol) was added to dichloromethane (5 ml), followed by adding triethylamine (1.05 g, 10.4 mmol) and 4-N,N-dimethylaminopyridine (0.13 g, 1.04 mmol), cooling to 0°C, adding p-toluenesulfonyl chloride (1.09 g, 5.7 mmol), and then reacting under stirring at room temperature overnight. The obtained reaction solution was diluted with dichloromethane (20 ml), silica gel (20 ml), and concentrated. The residue was separated and purified by a silica gel column (petroleum ether: ethyl acetate (V/V) = 2:1) to obtain white solid compound 1-(5-isopropylpyridin-2-yl)azetidin-3-yl 4-methylbenzenesulfonate **(032G)** (1.6 g, yield 89%).

LC-MS, M/Z (ESI): 347.44 (M+1)

Step 6: Synthesis of ((S)-2,2-dimethyl-1,3-dioxolan-4-yl)((3S,4S)-3-((R)-1-hydroxyethyl)-4-(3-((1-(5-isopropylpyridin-2-yl)azetidin-3-yl)oxy)-4-methoxyphenyl)-3-methylpyrrolidin-1-yl)methanone (**032H**)

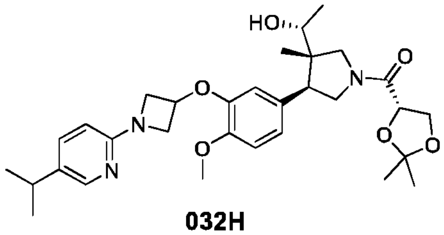

**032H**

The compound (S)-2,2-dimethyl-1,3-dioxolan-4-yl)((3S,4S)-4-(3-hydroxy-4-methoxyphenyl)-3-((R)-1-hydroxyethyl)-3-methylpyrrolidin-1-yl)methanone (**032D**) (0.4 g, 1.05 mmol) was added to DMF (4 ml), followed by adding cesium carbonate (0.52 g, 1.58 mmol), heating to 50°C and stirring for 0.5 h. Then 1-(5-isopropylpyridin-2-yl)azetidin-3-yl 4-methylbenzenesulfonate (**032G**) (0.37 g, 1.05 mmol) was added, heated to 90°C, and stirred for 4 h. The obtained solution was cooled to room temperature, diluted with distilled water (10 ml), and extracted with ethyl acetate (10 ml×3). The organic phases were combined, washed with saturated brine (10 ml×2), and separated. The separated organic phase was dried over sodium sulfate, filtered, and concentrate. The residue was purified with a silica gel column (petroleum ether: ethyl acetate (V/V) = 2:1) to obtain an oily compound ((S)-2,2-dimethyl-1,3-dioxolan-4-yl)((3S,4S)-3-((R)-1-hydroxyethyl)-4-(3-((1-(5-isopropylpyridin-2-yl)azetidin-3-yl)oxy)-4-methoxyphenyl)-3-methylpyrrolidin-1-yl)methanone (**032H**) (0.3 g, yield 51.4%).

LC-MS, M/Z (ESI): 554.70 (M+1).

Step 7: Synthesis of (S)-2,3-dihydroxy-1-((3S,4S)-3-((R)-1-hydroxyethyl)-4-(3-((1-(5-isopropylpyridin-2-yl)azetidin-3-yl)oxy)-4-methoxyphenyl)-3-methylpyrrolidin-1-yl)propan-1-one (**target compound 032**)

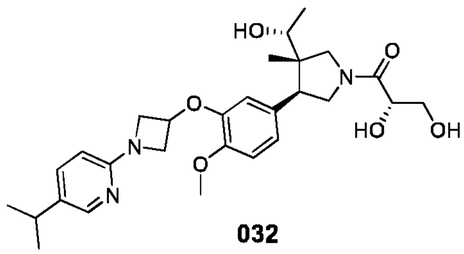

**032**

((S)-2,2-dimethyl-1,3-dioxolan-4-yl)((3S,4S)-3-((R)-1-hydroxyethyl)-4-(3-((1-(5-isopropylpyridin-2-yl)azetidin-3-yl)oxy)-4-methoxyphenyl)-3-methylpyrrolidin-1-yl)methanone (**032H**) (0.3 g, 0.54 mmol) was dissolved in dichloromethane (3 mL), 4N hydrogen chloride/1,4-dioxane solution (1 mL) was added, and the mixture was stirred at room temperature for 3 h. After the completion of the reaction, pH was adjusted to 8-9 with a saturated aqueous solution of sodium bicarbonate, and extraction was performed with dichloromethane (15 mL×3). The organic phases were combined, dried with anhydrous sodium sulfate, filtered, and concentrated. The residue was separated and purified with a silica gel column (pure ethyl acetate) to obtain the target compound, (S)-2,3-dihydroxy-1-((3S,4S)-3-((R)-1-hydroxyethyl)-4-(3-((1-(5-isopropylpyridin-2-yl)azetidin-3-yl)oxy)-4-methoxyphenyl)-3-methylpyrrolidin-1-yl)propan-1-one (**target compound 032**) (0.2 g, yield 71.9%).

$^1$H NMR (400 MHz, DMSO-d6) δ7.94 (s, 1H), 7.47-7.44 (dd, 1H), 6.93-6.86 (m, 2H), 6.67 (q, 1H), 6.42 (d, 1H), 5.11-5.07 (m,1H),4.91-4.70 (m, 3H), 4.34-4.31 (m, 3H), 3.83-3.73 (m, 3H), 3.64-3.46 (m, 9H), 3.16 (d, 1H), 2.79-2.76 (m, 1H), 1.14 (d, 6H), 0.99 (d, 3H), 0.62 (s, 3H).

LC-MS, M/Z (ESI): 514.63 (M+1).

Example 34: Preparation of target compound 033 (S)-2,3-dihydroxy-1-((3S,4S)-3-((R)-1-hydroxyethyl)-4-(4-methoxy-3-((1-(5-(2,2,2-trifluoroethyl)pyridin-2-yl)azetidin-3-yl)oxy)phenyl)-3-methylpyrrolidin-1-yl)propan-1-one (**target compound 033**)

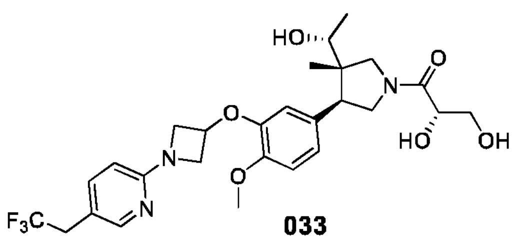

**033**

The specific synthesis scheme and method of compound 033 are as follows:

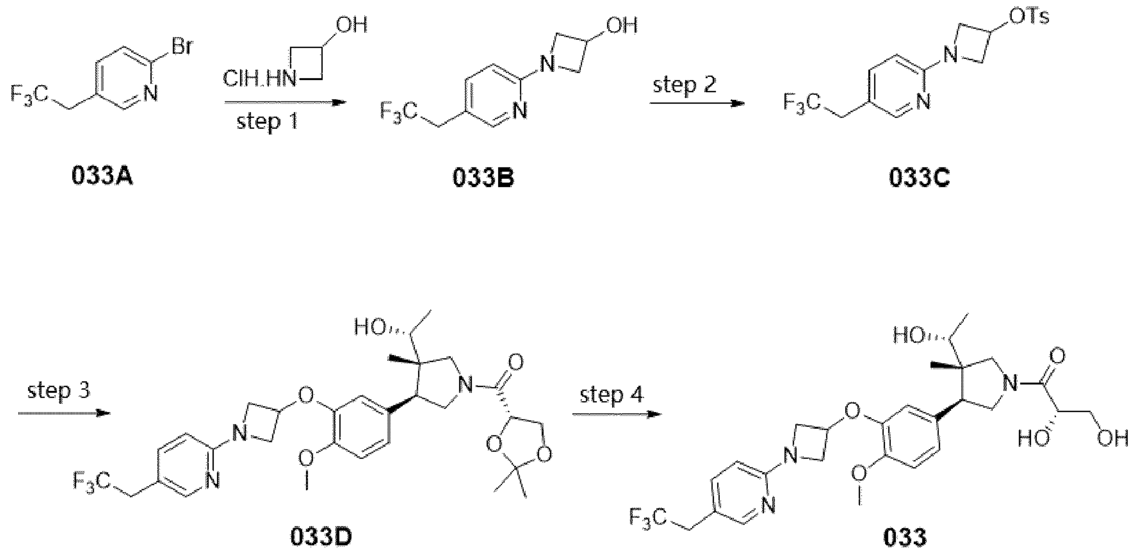


Step 1: Synthesis of 1-(5-(2,2,2-trifluoroethyl)pyridin-2-yl)azetidin-3-ol (**033B**)

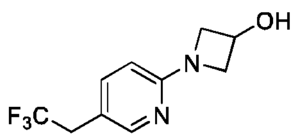

**033B**

At room temperature, 2-bromo-5-(2,2,2-trifluoroethyl)pyridine **(033A)** (1 g, 4.17 mmol) was added to 10 mL of DMSO, then 3-azetidinol hydrochloride (0.91 g, 8.33 mmol), cuprous iodide (0.79 g, 4.17 mmol), L-proline (0.1 g, 0.83 mmol) and cesium carbonate (3.39 g, 10.42 mmol) were added sequentially, and the reaction solution was heated to 95°C and stirred overnight under the protection of nitrogen. The reaction solution was cooled to room temperature, diluted with water (20 ml), and extracted with ethyl acetate (50 ml×3). The organic phases were combined, washed with saturated brine (50 ml×2), and separated. The separated organic phase was dried with anhydrous sodium sulfate, filtered, and concentrated. The residue was separated and purified with a silica gel column (petroleum ether: ethyl acetate (V/V) =2:1) to obtain white solid compound 1-(5-(2,2,2-trifluoroethyl)pyridin-2-yl)azetidin-3-ol (**033B**) (0.52 g, yield 53.8%).

LC-MS, M/Z (ESI): 232.21 (M+1)

Step 2: Synthesis of 1-(5-(2,2,2-trifluoroethyl)pyridin-2-yl)azetidin-3-yl 4-methylbenzenesulfonate (**033C**)

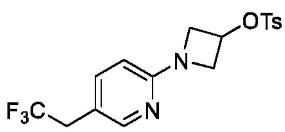

**033C**

1-(5-(2,2,2-trifluoroethyl)pyridin-2-yl)azetidin-3-ol (**033B**) (0.26 g, 1.12 mmol) was added to dichloromethane (5 ml), triethylamine (0.23 g, 2.24 mmol) and 4-N,N-dimethylaminopyridine (27 mg, 0.22 mmol) were added, the mixture was cooled to 0°C, p-toluenesulfonyl chloride (0.24 g, 1.23 mmol) was added, and the solution was stirred overnight at room temperature. The reaction solution was diluted with dichloromethane (10 mL), silica gel (5 ml), and concentrated. The residue was separated and purified by a silica gel column (petroleum ether: ethyl acetate (V/V) = 2:1) to obtain white

solid compound 1-(5-(2,2,2-trifluoroethyl)pyridin-2-yl)azetidin-3-yl 4-methylbenzenesulfonate **(033C)** (0.35 g, yield 81%).
LC-MS, M/Z (ESI): 387.39 (M+1)

Step 3: Synthesis of ((S)-2,2-dimethyl-1,3-dioxolan-4-yl)((3S,4S)-3-((R)-1-hydroxyethyl)-4-(4-methoxy-3-((1-(5-(2,2,2-trifluoroethyl)pyridin-2-yl)azetidin-3-yl)oxy)phenyl)-3-methylpyrrolidin-1-yl)methanone (**033D**)

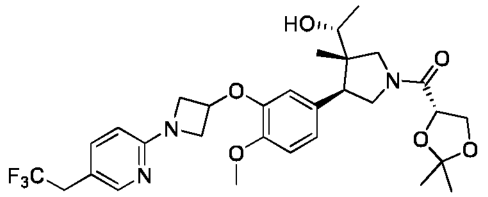

**033D**

The compound (S)-2,2-dimethyl-1,3-dioxolan-4-yl)((3S,4S)-4-(3-hydroxy-4-methoxyphenyl)-3-((R)-1-hydroxyethyl)-3-methylpyrrolidin-1-yl)methanone (**032D**, referring to the synthesis mentioned in step 3 of Example 33 of the present disclosure) (0.35 g, 0.92 mmol) was added to DMF (4 mL), cesium carbonate (0.45 g, 1.38 mmol) was added, and the mixture was heated to 50°C and stirred for 0.5 h. Then 1-(5-(2,2,2-trifluoroethyl)pyridin-2-yl)azetidin-3-yl 4-methylbenzenesulfonate (**033C**) (0.35 g, 0.92 mmol) was added. The solution was heated to 90°C, and stirred and reacted for 4 h. The obtained solution was cooled to room temperature, diluted with distilled water (10 mL), and extracted with ethyl acetate (10 mL×3). The organic phases were combined, washed with saturated brine (10 mL×2), and separated. The separated organic phase was dried with anhydrous sodium sulfate, filtered, and concentrated. The residue was purified with a silica gel column (petroleum ether: ethyl acetate (V/V) = 2:1) to obtain an oily compound ((S)-2,2-dimethyl-1,3-dioxolan-4-yl)((3S,4S)-3-((R)-1-hydroxyethyl)-4-(4-methoxy-3-((1-(5-(2,2,2-trifluoroethyl)pyridin-2-yl)azetidin-3-yl)oxy)phenyl)-3-methylpyrrolidin-1-yl)methanone (**033D**) (0.25 g, yield 45.7%).
LC-MS, M/Z (ESI): 594.64 (M+1).

Step 4: Synthesis of (S)-2,3-dihydroxy-1-((3S,4S)-3-((R)-1-hydroxyethyl)-4-(4-methoxy-3-((1-(5-(2,2,2-trifluoroethyl)pyridin-2-yl)azetidin-3-yl)oxy)phenyl)-3-methylpyrrolidin-1-yl)propan-1-one (**target compound 033**)

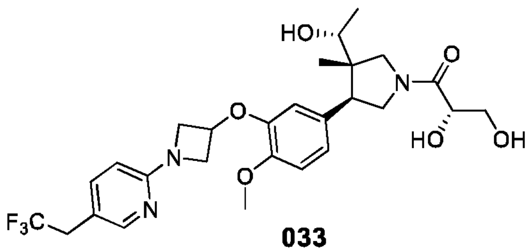

**033**

((S)-2,2-dimethyl-1,3-dioxolan-4-yl)((3S,4S)-3-((R)-1-hydroxyethyl)-4-(4-methoxy-3-((1-(5-(2,2,2-trifluoroethyl)pyridin-2-yl)azetidin-3-yl)oxy)phenyl)-3-methylpyrrolidin-1-yl)methanone (**033D**) (0.25 g, 0.42 mmol) was dissolved in dichloromethane (3 mL), 4N hydrogen chloride/1,4-dioxane solution (1 mL) was added, and the mixture was stirred at room temperature for 3 h. After the reaction was finished, the obtained solution was adjusted to pH of 8-9 with a saturated aqueous solution of sodium bicarbonate, and extracted with dichloromethane (15 mL×3). The organic phases were combined, dried with anhydrous sodium sulfate, filtered, and concentrated. The residue was separated and purified by a silica gel column (pure ethyl acetate) to obtain the target compound (S)-2,3-dihydroxy-1-((3S,4S)-3-((R)-1-hydroxyethyl)-4-(4-methoxy-3-((1-(5-(2,2,2-trifluoroethyl)pyridin-2-yl)azetidin-3-yl)oxy)phenyl)-3-methylpyrrolidin-1-yl)propan-1-one (**target compound 033**) (0.18 g, yield 77.2%).
$^1$H NMR (400 MHz, DMSO-d6) δ8.03 (s, 1H), 7.50-7.49 (m, 1H), 6.91-6.85 (m, 2H), 6.68 (S, 1H), 6.46 (d, 1H), 5.10-4.70 (m, 5H), 4.36-4.22 (m, 4H), 3.90-3.44 (m, 15H), 3.16 (d, 1H), 2.34-2.30 (m, 1H), 0.99 (d, 3H), 0.62 (s, 3H).
LC-MS, M/Z (ESI): 554.58 (M+1).

Example 35: Preparation of target compound 034 (S)-1-((3S,4S)-4-(3-((1-(5-cyclopropylpyridin-2-yl)azetidin-3-yl)oxy)-4-methoxyphenyl)-3-((R)-1-hydroxyethyl)-3-methylpyrrolidin-1-yl)-2,3-dihydroxypropan-1-one (**target compound 034**)

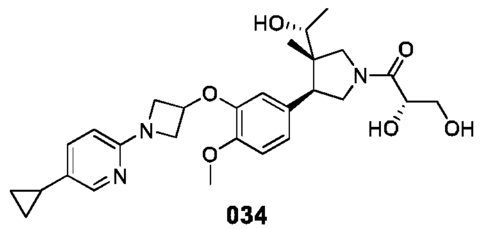

**034**

The specific synthesis scheme and method of compound 034 are as follows:

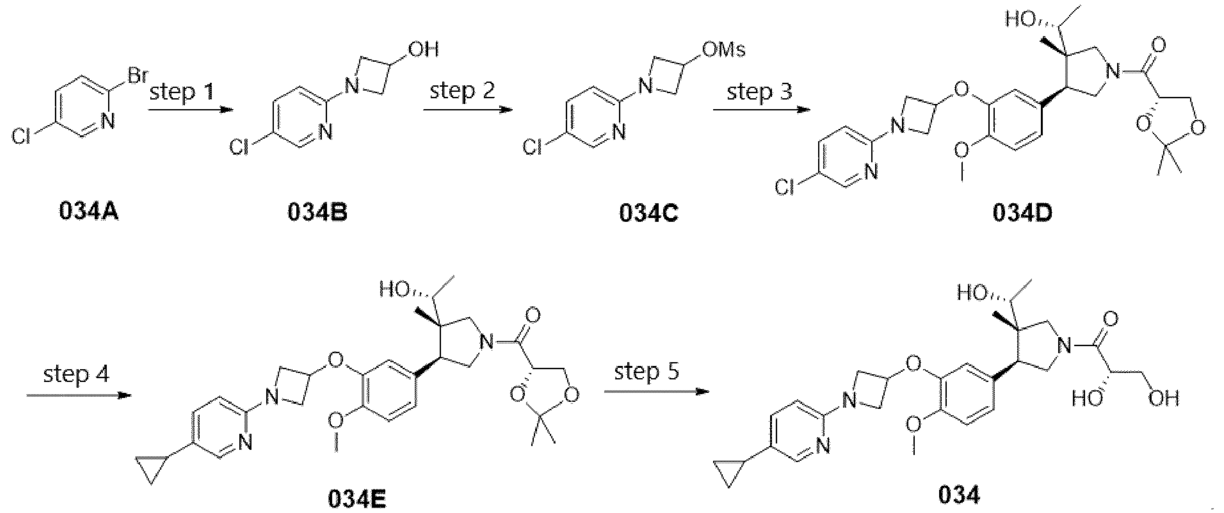


**034A** **034B** **034C** **034D** **034E** **034**

Step 1: Synthesis of 1-(5-chloropyridin-2-yl)azetidin-3-ol (**034B**)

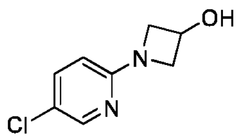

**034B**

At room temperature, 5-chloro-2-bromopyridine **(034A)** (25.0 g, 130.2 mmol) was added to DMSO (300mL), 3-azetidinol hydrochloride (17.0 g, 156.3 mmol), cuprous iodide (2.5 g, 13.0 mmol), L-proline (16.5 g, 143.2 mmol), and potassium phosphate (55.2 g, 260.4 mmol) were added, and the mixture was heated to 115°C under the protection of nitrogen and stirred for 16 h. The obtained solution was cooled to room temperature, diluted with water (1500 mL), extracted with ethyl acetate (500 mL×3), and separated. The organic phases were combined, dried with anhydrous sodium sulfate, filtered, and concentrated. The residue was separated and purified with a silica gel column (petroleum ether: ethyl acetate (V/V) = 2:1) to obtain light yellow liquid 1-(5-chloropyridin-2-yl)azetidin-3-ol (**034B**) (17.0 g, yield 70.9%).

LC-MS,M/Z (ESI): 184.8 (M+1).

Step 2: Synthesis of 1-(5-chloropyridin-2-yl)azetidin-3-yl methanesulfonate (**034C**)

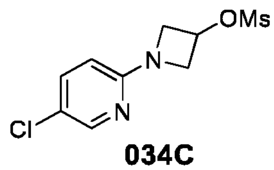

034C

1-(5-chloropyridin-2-yl)azetidin-3-ol **(034B)** (17.0 g, 92.4 mmol) was added to dichloromethane (200 mL) at room temperature, followed by adding triethylamine (18.7 g, 184.8 mmol), cooling to 0°C, adding methanesulfonyl chloride (12.6 g, 110.9 mmol), and stirring at room temperature for 3 h. The obtained solution was diluted with dichloromethane (200 mL), washed with water (60 mL×3), and separated. The separated organic phase was dried with anhydrous sodium sulfate, filtered, and concentrated. The residue was separated and purified with a silica gel column (petroleum ether: ethyl acetate (V/V) = 2:1) to obtain light yellow solid 1-(5-chloropyridin-2-yl)azetidin-3-yl methanesulfonate **(034C)** (22 g, yield 90.8%).

LC-MS,M/Z (ESI): 263.7 (M+1).

Step 3: Synthesis of ((3S,4S)-4-(3-((1-(5-chloropyridin-2-yl)azetidin-3-yl)oxy)-4-methoxyphenyl)-3-((R)-1-hydroxyethyl)-3-methylpyrrolidin-1-yl)((S)-2,2-dimethyl-1,3-dioxolan-4-yl)methanone (**034D**)

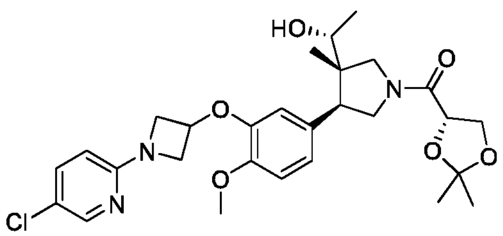

034D

The compound (S)-2,2-dimethyl-1,3-dioxolan-4-yl)((3S,4S)-4-(3-hydroxy-4-methoxyphenyl)-3-((R)-1-hydroxyethyl)-3-methylpyrrolidin-1-yl)methanone (**032D**) (6.0 g, 15.8 mmol) was added to DMF (50 mL), and potassium phosphate (10.1 g, 47.4 mmol) was added. The mixture was heated to 90°C, stirred for 0.5 h, and then cooled to 40°C, followed by adding 1-(5-chloropyridin-2-yl)azetidin-3-yl methanesulfonate **(034C)** (5.0 g, 18.9 mmol), heating to 90°C, and stirring for 16 h. The obtained solution was cooled to room temperature, diluted with water (200 mL), extracted with ethyl acetate (200 mL×3), and separated. The organic phases were combined, dried with anhydrous sodium sulfate, filtered, and concentrated. The residue was purified with a silica gel column (petroleum ether: ethyl acetate (V/V) = 2:1) to obtain white solid ((3S,4S)-4-(3-((1-(5-chloropyridin-2-yl)azetidin-3-yl)oxy)-4-methoxyphenyl)-3-((R)-1-hydroxyethyl)-3-methylpyrrolidin-1-yl)((S)-2,2-dimethyl-1,3-dioxolan-4-yl)methanone (**034D**) (4.3g, yield 49.7%).

LC-MS, M/Z (ESI): 546.3 (M+1).

Step 4: Synthesis of ((3S,4S)-4-(3-((1-(5-cyclopropylpyridin-2-yl)azetidin-3-yl)oxy)-4-methoxyphenyl)-3-((R)-1-hydroxyethyl)-3-methylpyrrolidin-1-yl)((S)-2,2-dimethyl-1,3-dioxolan-4-yl)methanone (**034E**)

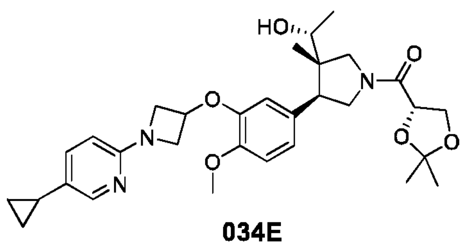

034E

The raw material ((3S,4S)-4-(3-((1-(5-chloropyridin-2-yl)azetidin-3-yl)oxy)-4-methoxyphenyl)-3-((R)-1-hydroxyethyl)-3-methylpyrrolidin-1-yl)((S)-2,2-dimethyl-1,3-dioxolan-4-yl)methanone (**034D**) (546 mg, 1.0 mmol) was added to

toluene (20 mL) at room temperature, and water (1 mL) was added. Then, bis (tri-tert-butylphosphine)palladium (51 mg, 0.1 mmol) and potassium phosphate (424 mg, 2.0 mmol) were added under the protection of nitrogen, heated to 110°C, and stirred for 16 h. The obtained solution was cooled to room temperature, diluted with water (20 mL), extracted with ethyl acetate (20 mL×3), and separated. The organic phases were combined, dried with anhydrous sodium sulfate, filtered, and concentrated. The residue was purified with a silica gel column (petroleum ether: ethyl acetate (V/V) = 2:1) to obtain white solid crude product ((3S,4S)-4-(3-((1-(5-cyclopropylpyridin-2-yl)azetidin-3-yl)oxy)-4-methoxyphenyl)-3-((R)-1-hydroxyethyl)-3-methylpyrrolidin-1-yl)((S)-2,2-dimethyl-1,3-dioxolan-4-yl)methanone (**034E**) (220 mg, yield 39.8%).

LC-MS, M/Z (ESI): 552.4 (M+1)

Step 5: Synthesis of (S)-1-((3S,4S)-4-(3-((1-(5-cyclopropylpyridin-2-yl)azetidin-3-yl)oxy)-4-methoxyphenyl)-3-((R)-1-hydroxyethyl)-3-methylpyrrolidin-1-yl)-2,3-dihydroxypropan-1-one (**034**)

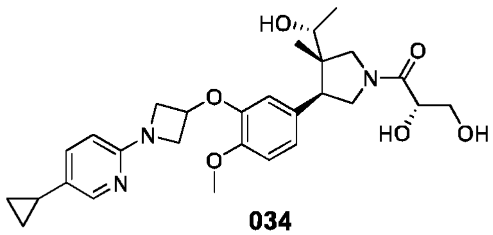

**034**

((3S,4S)-4-(3-((1-(5-cyclopropylpyridin-2-yl)azetidin-3-yl)oxy)-4-methoxyphenyl)-3-((R)-1-hydroxyethyl)-3-methylpyrrolidin-1-yl)((S)-2,2-dimethyl-1,3-dioxolan-4-yl)methanone (**034E**) (150 mg, 0.27 mmol) was dissolved in tetrahydrofuran (4 mL), followed by adding IN hydrochloric acid (2 mL) and stirring at room temperature for 16 h. The reaction solution was concentrated to prepare white solid (S)-1-((3S,4S)-4-(3-((1-(5-cyclopropylpyridin-2-yl)azetidin-3-yl)oxy)-4-methoxyphenyl)-3-((R)-1-hydroxyethyl)-3-methylpyrrolidin-1-yl)-2,3-dihydroxypropan-1-one (**034**) (17 mg, 12.2% yield).

$^1$H NMR (400m Hz, DMSO-d6) δ7.94 (d, 1H), 7.24 (d, 1H), 6.92-6.97 (m, 2H), 6.68 (d, 1H), 6.39 (d, 1 H), 5.11-5.09 (m, 1H), 4.33-4.31 (m, 2H), 4.27-4.21 (m, 1H), 3.95-3.91 (m, 1H), 3.84-3.81 (m, 2H), 3.74 (s, 3H), 3.65-3.42 (m, 8H), 3.36-3.34 (m, 2H), 1.83-1.77 (m, 1H), 1.01 (d, 3H), 0.86-0.84 (m, 2H), 0.64 (s, 3H), 0.57-0.55 (m, 2H).

LC-MS, M/Z (ESI): 512.7 (M+1).

**Test examples regarding biological activity and related properties**

Test example 1: PDE4B and PDE4D enzyme activity assay

For the previously synthesized compounds, the inhibitory activity of the compounds against PDE4B and against PDE4D was tested by using PDE-Glo Phosphodiesterase Assay Kit (promega, V1361). In short, in accordance with the instructions of the kit, a concentrated stock solution (10 mM) the compound to be tested was first prepared with DMSO solvent, and the concentrated stock solution was then diluted into 10x working solutions with a Reaction buffer provided in the kit. With the Reaction buffer, on ice, PDE4B enzyme (Enzo Life Sciences, BML-SE522-0020) was diluted to a concentration of 1 ng/μl to obtain a PDE4B enzyme working solution, and PDE4D enzyme (Enzo Life Sciences, BML-SE523-0020) was diluted to a concentration of 4 ng/μl to obtain a PDE4D enzyme working solution. 1.5 μl of the PDE4B working solution (or PDE4D working solution) and 1 μl of the compound working solution were added to wells of 384-well plate (Corning, CLS3707), and incubated with shaking at room temperature for 5 min, followed by adding 2.5 μl of cAMP (in Reaction buffer, 2μM) per well, incubating with shaking at room temperature for 20 min. 2.5μl/well of 1×Termination Buffer, and then 2.5μl/well of 1×Detection Buffer were added, and further incubated with shaking at room temperature for 20 min. 10μl/well of 1×Kinase-Glo was added, and the plate was incubated with shaking at room temperature for 10 min, and bioluminescence was measured with PheraStar instrument. The experimental results were plotted with GraphPad Prism software, and fitted to calculate IC50 of each compound.

The experimental results indicate that, compared with the positive control compound, the compounds of the present disclosure, such as compounds 001 to 031, all have better or comparable inhibitory activity against PDE4. In addition, through the comparison assay of the inhibitory activity of PDE4B/PDE4D, it was found that, compared with the closest prior art (the positive control compound), the compounds of the present disclosure, such as compounds 001 to 004, 006 to 012, 014 to 015, 025, 027, 028, and 031 to 034, exhibit better selective inhibition against PDE4B, and can

effectively alleviate or eliminate the occurrence of side effects such as vomiting. Further, according to the embodiments of the present disclosure, the test results of compounds 005, 013, 016, 017, 018, 024, 029 and 030 indicate that all these compounds can effectively inhibit PDE4B, and when compared with the selective inhibitory activity against PDE4D, the selective inhibitory activity against PDE4B of these compounds is significantly higher than that control compound.

The data of some results are summarized in the following table to facilitate understanding.

[Table 1] Results of inhibitory activity of test compounds against PDE4B and PDE4D

| Test compound | PDE4B $IC_{50}$ (nM) | PDE4D $IC_{50}$ (nM) | $IC_{50}$ (PDE4D)/$IC_{50}$ (PDE4B) |
|---|---|---|---|
| Positive control compound | 2.10 | 0.41 | 0.20 |
| 001 | 0.91 | 0.67 | 0.74 |
| 002 | 0.43 | 0.16 | 0.37 |
| 003 | 0.41 | 0.39 | 0.95 |
| 004 | 0.93 | 1.12 | 1.20 |
| 006 | 1.37 | 1.34 | 0.98 |
| 007 | 1.17 | 3.94 | 3.37 |
| 008 | 1.46 | 3.58 | 2.45 |
| 009 | 0.78 | 4.67 | 5.99 |
| 010 | 1.90 | 0.95 | 0.50 |
| 011 | 2.31 | 1.07 | 0.46 |
| 012 | 2.09 | 0.56 | 0.27 |
| 014 | 6.65 | 6.02 | 0.91 |
| 015 | 0.68 | 0.87 | 1.28 |
| 019 | 1.87 | | |
| 020 | 1.97 | | |
| 021 | 4.15 | 14.9 | 3.59 |
| 022 | 0.75 | 1.18 | 1.57 |
| 023 | 1.30 | | |
| 025 | 0.88 | 2.16 | 2.45 |
| 026 | 2.76 | | |
| 027 | 14.32 | 24.81 | 1.73 |
| 028 | 2.22 | 3.08 | 1.39 |
| 031 | 1.87 | 2.56 | 1.37 |
| 032 | 0.33 | 3.25 | 9.85 |
| 033 | 0.22 | 2.81 | 12.8 |
| 034 | 0.25 | 2.96 | 11.8 |

Test example 2: Model test on LPS-induced secretion of TNF$\alpha$ in human PBMCs

Extraction process of peripheral blood mononuclear cells (PBMCs): one unit of human peripheral blood concentrate (concentrated from 200cc peripheral blood) was sucked and added with 0.9% saline until a total volume reached 120ml, and mixed homogenously. 15ml Lymphoprep™ was added into 50ml centrifuge tube, and then 30ml of diluted blood concentrate was carefully and slowly added thereto, allowing the diluted blood to be stacked on the layered solution and preventing the diluted blood from being mixed into the separated solutions or breaking the surface of the separated solution, wherein a ratio of Lymphoprep™ to the diluted blood was 1:2. The centrifuge tubes were balanced and placed in a horizontal centrifuge (eppendorf, 5810R), and centrifuged at 800g and 20°C for 20 min. The centrifuge tubes were

carefully taken out. PBMCs were absorbed by directly inserting the Pasteur pipette deep into the buffy coat. The obtained PBMCs were gently pipetted with 3 times the volume of 0.9% normal saline or PBS (without calcium or magnesium ions) to mix homogenously. After that, the cells were centrifuged at 250g and 20°C for 10 min, the supernatant was removed to remove the platelets remaining in the cell suspension. The obtained cell pellet was resuspended in 20ml of PBS, and counted using trypan blue.

Compound screening by using PBMCs: the PBMC suspension obtained above was centrifuged to remove PBS, then resuspended in complete medium (RPMI1640 + 10% FBS + 1% P/S) and counted. 100 μL/well of the cells was seeded at $5 \times 10^4$/well. Each of the compounds to be screened was prepared as 4 times the final concentration, which was added to the cells at 50 μL/well. The compound was pre-incubated for 30 min in advance of being added to the cells. Meanwhile, control wells to which the compound was not added were provided. A final stimulation concentration of LPS was 10 ng/ml, and LPS was formulated as 4 times the final stimulation concentration, and added to the cells at 50 μL/well. Meanwhile, control wells, i.e., wells without LPS were provided. The cells were incubated for 24 h, and 10% of the supernatant was collected for detection. The obtained supernatant was detected using Invitrogen's Human TNF-α kit (REF: 88-7346-88).

The experimental results indicate that, compared with the positive control, the compounds of the present disclosure have better or comparable inhibitory activity against TNFα secreted by human PBMCs, and especially, the effects of the compounds 001 to 003, 006 to 008, 010 to 012, 028, and 031 to 034 are more superior than the positive control, thereby significantly inhibiting the secretion of the inflammatory factor TNFα in human PBMCs, and having a more prominent anti-inflammatory effect.

The data of some results are summarized in the following table to facilitate understanding.

[Table 2] Results of inhibitory activity of test compounds on LPS-induced secretion of TNFα in human PBMCs

| Test compound | $IC_{50}$ (nM) |
|---|---|
| Positive control compound | 3.32 |
| 001 | 0.36 |
| 002 | 0.56 |
| 003 | 1.48 |
| 004 | 5.13 |
| 005 | 6.18 |
| 006 | 2.52 |
| 007 | 2.24 |
| 008 | 1.81 |
| 009 | 7.57 |
| 010 | 1.05 |
| 011 | 1.55 |
| 012 | 2.52 |
| 014 | 15.1 |
| 015 | 3.34 |
| 020 | 9.38 |
| 021 | 14.1 |
| 022 | 1.01 |
| 023 | 5.4 |
| 024 | 6.56 |
| 025 | 3.26 |
| 026 | 14.9 |
| 028 | 2.94 |

(continued)

| Test compound | $IC_{50}$ (nM) |
|---|---|
| 031 | 0.70 |
| 032 | 0.26 |
| 033 | 0.53 |
| 034 | 0.32 |

Test Example 3: Determination of LPS-stimulated TNF-α release in mice

18 Balb/c mice (6 to 8 weeks) were randomly assigned to a blank group, a model group, and an administration group, 6 mice in each group. A vehicle (blank group, model group) or 50 mg/kg compound (administration group, where the dose for the positive control group was 100 mg/kg) was orally administrated. 30 min after the oral administration, PBS (blank group) or 1mg/kg LPS (model group and administration group) was injected intraperitoneally. 90 min or 120 min after the intraperitoneal injection, blood was collected from the hearts of the mice, and still stood at 2-8°C for 4 hours, and centrifuged at 5000 rpm for 10 min to collect the serum. The serum was stored at -80°C for testing.
The serum TNF-α level was tested using a mouse TNF-α detection kit (Mouse TNFa ELISA kit: Biolegend, Cat: 430904). The inhibitory activity of the compounds against the TNF-α release was calculated based on the serum TNF-α level.
Inhibitory activity of compound %={1 - (TNFα concentration of administration group - TNFα concentration of blank group)/(TNFα concentration of model group - TNFα concentration of blank group)}×100%.
Experimental results showed that, compared with the positive control group, the compounds of the present disclosure can better inhibit the LPS-stimulated TNF-α release in mice, and have significantly higher inhibitory activity than the positive control group, thereby having more prominent anti-inflammatory effect.
The data of some results are summarized in the following table to facilitate understanding.

Table 3: Determination of LPS-stimulated TNF-α release in mice

| | Inhibition rate (%) | |
|---|---|---|
| | 90 min | 120 min |
| Positive control compound | 44.4 | 49.0 |
| 001 | 53.6 | 39.5 |
| 002 | | 66.2 |
| 004 | 39.6 | 58.6 |
| 007 | 52.1 | 66.4 |
| 008 | | 63 |
| 009 | | 47.6 |
| 010 | 47.6 | 65.4 |
| 011 | 47.4 | 63.3 |
| 022 | 60.7 | |
| 031 | 64.8 | 78.3 |
| 032 | 75.8 | 84.0 |
| 033 | 70.3 | 75.9 |
| 034 | 76.4 | 85.6 |

Test Example 4: Pharmacokinetic test

In the rat pharmacokinetic test, 3 male SD rats (180-240g) were fasted overnight, and orally administered with 10 mg/kg of compound to be tested. Blood was collected before the administration, and 15 min, 30 min, 1h, 2h, 4h, 8h,

and 24h hours after the administration. The blood sample was centrifuged at 8000 rpm for 6 min at 4°C, and the plasma was collected and stored at -20°C. The plasma collected at each time point was added and mixed with 3-5 times the amount of acetonitrile solution containing an internal standard, vortexed for 1 min, centrifuged at 13,000 rpm for 10 min at 4°C. The supernatant was taken and mixed with 3 times the amount of water, and an appropriate amount of the mixture was taken for LC-MS/MS analysis. The main pharmacokinetic parameters were analyzed with WinNonlin 7.0 software non-compartmental model.

In the mouse pharmacokinetic test, 9 male CD-1 mice (20-25 g) were fasted overnight, and orally administered with 10 mg/kg of the compound to be tested. Bloods of 3 mice were collected at each time point, and bloods of 9 mice were alternately collected in total. In the pharmacokinetic test in canine, 3 male beagle dogs (8-10 kg) were fasted overnight, and orally administered with 5 mg/kg of the compound to be test. In the pharmacokinetic test of cynomolgus monkeys, 3 male cynomolgus monkeys (4 to 6.5 kg) were fasted overnight, and orally administered 20 mg/kg of the compound to be test. The remaining processes are the same as those of the rat pharmacokinetic test.

[Table 4] Results of pharmacokinetic test of mice, rats and canines

| Test compound | Pharmacokinetic parameters (oral gavage administration) | | | | | | | | |
|---|---|---|---|---|---|---|---|---|---|
| | Mouse | | | Rat | | | Canine | | |
| | Cmax (ng/mL) | Tmax (hr) | AUCO-t (h*ng/mL) | Cmax (ng/ mL ) | Tmax (hr) | AUCO-t (h*ng/mL) | Cmax (ng/mL) | Tmax (hr) | AUCO-t (h*ng/m L) |
| Positive control compound | 142.3 | 0.25 | 237.4 | 68.2 | 0.5 | 189.6 | 159 | 0.92 | 423 |
| 001 | 339.2 | 0.25 | 311.2 | 22.5 | 0.33 | 23.5 | | | |
| 002 | 354.6 | 0.25 | 386 | 41.6 | 0.33 | 102.9 | 197.6 | 1 | 534 |
| 004 | 378 | 0.5 | 896 | 823.3 | 0.42 | 2482 | 484 | 0.83 | 1738 |
| 007 | 125 | 0.5 | 332 | 735 | 0.5 | 1532 | 689 | 0.25 | 1687 |
| 008 | 298 | 0.5 | 625 | 133 | 0.25 | 442.7 | 534 | 0.5 | 1755 |
| 010 | 314 | 0.25 | 645 | 40.4 | 0.33 | 95.3 | 413 | 0.83 | 1639 |
| 011 | 316 | 0.5 | 1054 | 126 | 0.42 | 294 | 356 | 0.83 | 1423 |
| 022 | 186 | 0.25 | 215 | 221 | 0.33 | 410 | | | |

[Table 5] Results of pharmacokinetic test of cynomolgus monkey

| Test compound | Pharmacokinetic parameters of cynomolgus monkeys (oral gavage administration) | | |
|---|---|---|---|
| | Cmax (ng/mL) | Tmax (hr) | AUCO-t (h*ng/mL) |
| Positive control compound | 565 | 1.50 | 3640 |
| 007 | 13843 | 1.75 | 44400 |

The experimental results in Table 4 and Table 5 indicate that, compared with the positive control group, the compounds of the present disclosure have better exposure and exhibit better pharmacokinetic properties. Moreover, Applicant also investigated the bioavailability, which was significantly better than the control compound.

Test Example 5: Mouse CIA Arthritis model test

**1. Experimental process**

**(1) Preparation of type II collagen/complete Freund's adjuvant**

**Preparation of acetic acid:** 2N acetic acid was diluted to 100 mM, filtered with 0.22 μm filter membrane, and stored at 4°C.

**Preparation of bovine type II collagen solution:** bovine type II collagen (CII) was dissolved in 100 mM acetic acid solution and stored at 4°C overnight. A final concentration of collagen was 8 mg/mL.

**Preparation of emulsion:** the CII solution stored overnight was mixed with an equal volume of complete Freund's adjuvant, and homogenized on ice by a high-speed homogenizer at 30,000 rpm for approximately 60 min, until the solution formed a stable emulsion.

**(2) Induction of arthritis:**

After DBA/1 mice were anesthetized with isoflurane, 50 μl of prepared collagen emulsion (containing 200 micrograms of CII) was injected subcutaneously in the tail (2-3 cm from the base of the tail) for immunization. The day of the primary immunization was recorded as day 0, and the following days were recorded sequentially. On day 21, the same volume of collagen emulsion was injected into the tail in the same way. Mice in the normal group were not immunized.

**(3) Administration and dosage**

On day 21, the mice that had been induced to have arthritis were randomly grouped according to their body weights, such that the average body weight of each group was consistent, and they were randomly divided into 6 treatment groups for administration, with 10 mice in each group.

G1 group included normal mice without being treated; G2 group was given a blank vehicle; G3 to G7 groups were administrated with the compound at a dose of 30 mg/kg, twice a day for 21 days. The volume of oral administration was 10 mL/kg.

**(4) Determination of incidence indexes of arthritis**

After the enhanced immunization, the disease incidence of the mice was observed every day. When the mice began to develop symptoms (clinical symptoms of arthritis emerged), the symptoms were graded as 0 to 4 according to the different degrees of pathological changes (redness, joint deformation), where the highest score for each limb was 4, and the highest score for each animal was 16. The mice were graded at least three times a week.

The area under curve (AUC) of clinical scores of each group from day 21 to day 42 was calculated, and the inhibition rate % of the clinical score AUC of the administration group was calculated:

$$\text{Inhibition rate \%}=1-(\text{administration group AUC}/\text{blank vehicle group AUC})*100\%$$

**(5) Statistical disposition**

The experimental data are expressed as Mean ± standard error (Mean ± SEM), and the clinical scores were analyzed with One-way ANOVA, where $p < 0.05$ was considered to be a significant difference.

**2. Experimental results**

Referring to FIG. 1, the results of the clinical scores and inhibition rates based on the clinical score AUC of each group at the experiment endpoint are shown in Table 6 below.

[Table 6]

| Group | No. | Clinical score (endpoint) | inhibition rate based on clinical score AUC |
|---|---|---|---|
| G1 | Normal mouse | 0 | |
| G2 | Blank vehicle | 8.5 | |
| G3 | Positive control compound | 4.4 | 42.3% |
| G4 | 004 | 3.3 | 67.5% |
| G5 | 011 | 4.8 | 49.3% |
| G6 | 007 | 3.1 | 77.2% |
| G7 | 031 | 2.1 | 85.0% |

At the experiment endpoint (day 42), the clinical score of each administration group was significantly lower than that of the blank vehicle group. The results regarding the inhibition rates based on the clinical score AUC from day 21 to day 42 indicate that, compared with the positive control group, the compounds of the present disclosure exhibit better inhibitory efficacies against arthritis.

In the specification, descriptions with reference to the terms "an embodiment", "some embodiments", "examples", "specific examples", or "some examples", etc. mean specific features, structures, materials or characteristics described in conjunction with the embodiment or example are included in at least one embodiment or example of the present disclosure. In this specification, the above terms are illustrative, and do not necessarily refer to the same embodiment or example. Moreover, the described specific features, structures, materials or characteristics can be combined in a suitable manner in any one or more embodiments or examples. In addition, those skilled in the art can combine the different embodiments or examples and the features of the different embodiments or examples described in this specification without contradicting each other.

Although the embodiments of the present disclosure are illustrated and described above, it can be understood that the above-mentioned embodiments are illustrative and should not be construed as limitations of the present disclosure. Those skilled in the art can make changes, modifications, substitutions, and variations based on the above-mentioned embodiments within the scope of the present disclosure.

## Claims

1. A compound, being a compound represented by formula (A); or being a tautomer, a stereoisomer, a hydrate, a solvate, a salt, or a prodrug of the compound represented by formula (A):

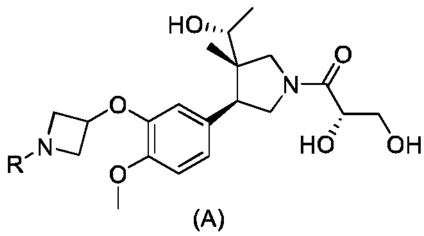

(A)

wherein,

R is

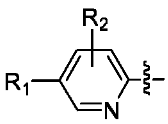, 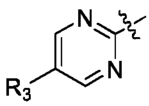, 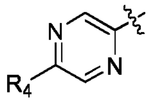, or 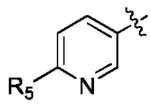;

$R_1$ and $R_2$ are each independently selected from H, $C_1$-$C_{10}$ alkyl, $C_3$-$C_{10}$ cycloalkyl, $C_1$-$C_{10}$ alkoxyl, halogen, and -CN; and
$R_3$, $R_4$, and $R_5$ are each independently selected from $C_1$-$C_{10}$ alkyl, $C_3$-$C_{10}$ cycloalkyl, $C_1$-$C_{10}$ alkoxyl, halogen, and -CN,
under a premise that:

when $R_1$ is $-CH_3$, C1, or -CN, $R_2$ is not H, and
when $R_1$ is H, $R_2$ is not H, -CN, or $-CH_3$.

2. The compound according to claim 1, wherein $R_1$, $R_3$, $R_4$, and $R_5$ are each independently selected from halogen, $C_1$-$C_5$ alkyl, $C_1$-$C_5$ alkoxyl, halogen, and -CN.

3. The compound according to claim 3, wherein $R_1$, $R_3$, $R_4$, and $R_5$ are each independently selected from halogen, $C_1$-$C_3$ alkyl, $C_1$-$C_3$ alkoxyl, halogen, and -CN.

4. The compound according to claim 1, wherein $R_2$ is selected from halogen, $C_1$-$C_5$ alkyl, $C_1$-$C_5$ alkoxyl, halogen, and

-CN.

**5.** The compound according to claim 4, wherein $R_2$ is selected from halogen, $C_1$-$C_3$ alkyl, $C_1$-$C_3$ alkoxyl, halogen, and -CN.

**6.** The compound according to claim 1, wherein the halogen is F, Cl, Br, or I.

**7.** The compound according to claim 1, wherein

$R_1$ is selected from $-CH_3$, $-CF_3$, $-CH_2CH_3$, $-CH_2CF_3$, $-OCH_3$, $-OCH_2CH_3$, $-OCH(CH_3)_2$, cyclopropyl, F, Cl, Br, or -CN;
$R_2$ is selected from H, $-CH_3$, $-CH_2CH_3$, F, Cl, -CN; or
$R_3$, $R_4$ and $R_5$ are each independently selected from $-CH_3$, $-CF_3$, $-CH_2CH_3$, $-CH_2CF_3$, - $OCH_3$, $-OCH_2CH_3$, $-OCH(CH_3)_2$, cyclopropyl, F, Cl, Br, or -CN;

**8.** The compound according to claim 1, wherein the compound is any one of the following compounds; or the compound is a tautomer, a stereoisomer, a hydrate, a solvate, a pharmaceutically acceptable salt, or a prodrug of any one of the following compounds:

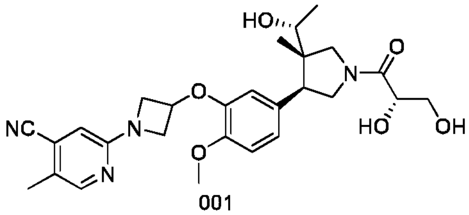

001

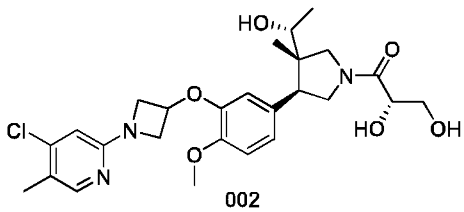

002

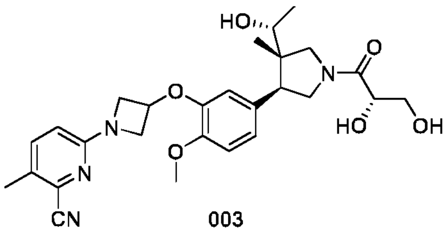

003

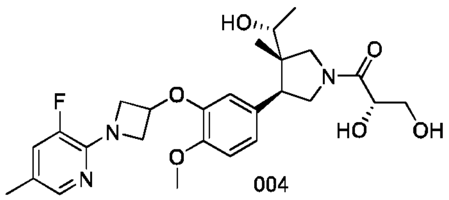

004

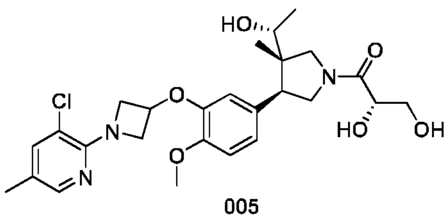

005

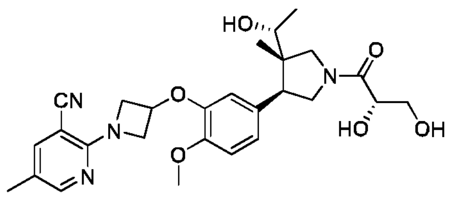

006

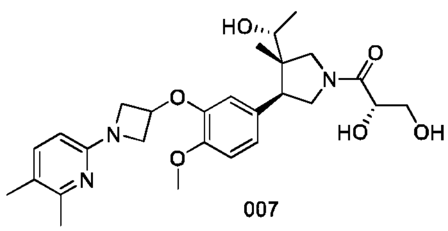

007

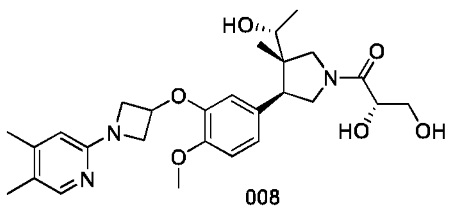

008

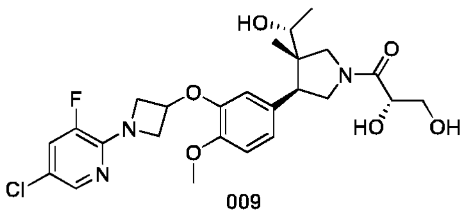

009

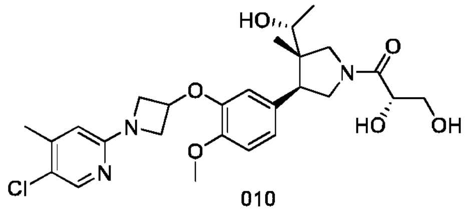

010

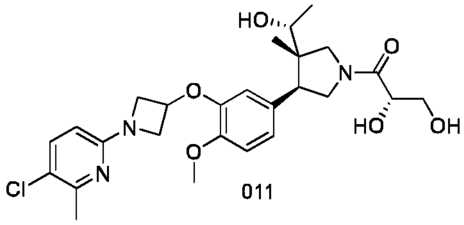

011

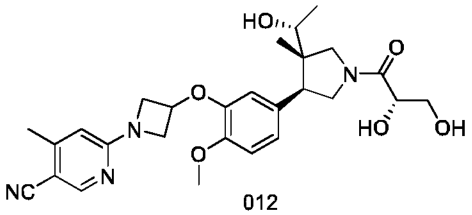

012

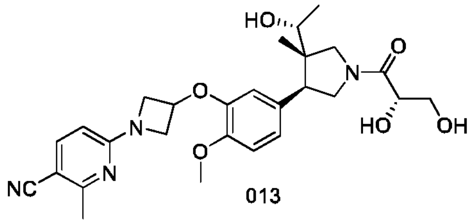

013

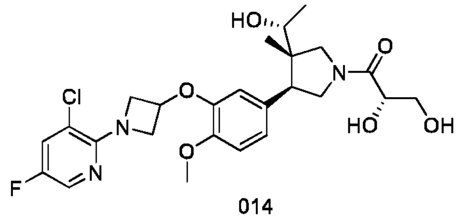

014

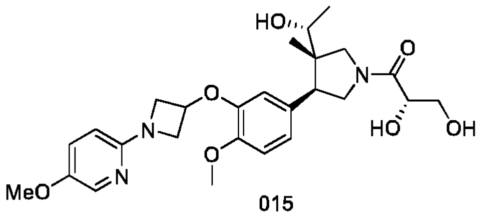

015

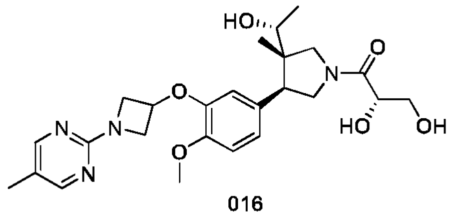

016

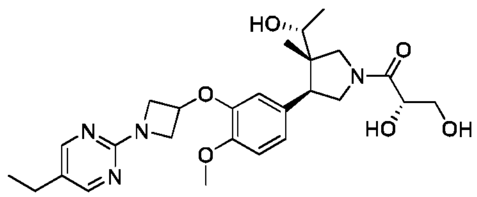

017

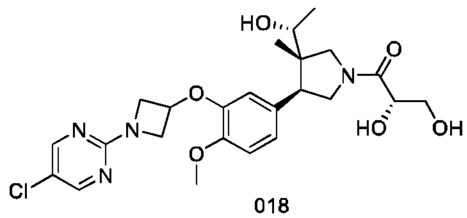

018

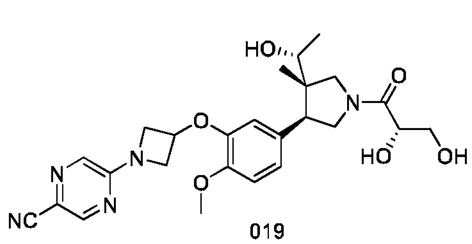

019

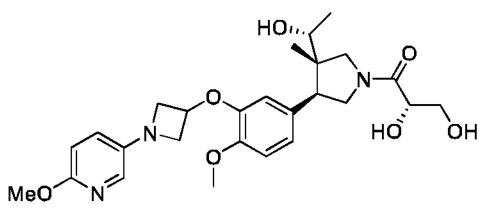

020

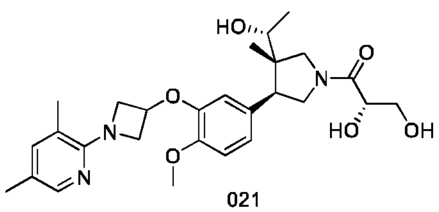

021

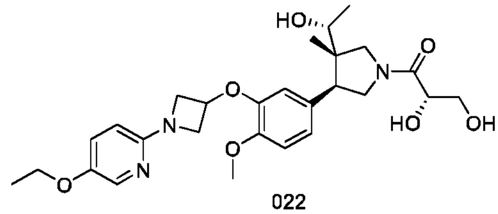

022

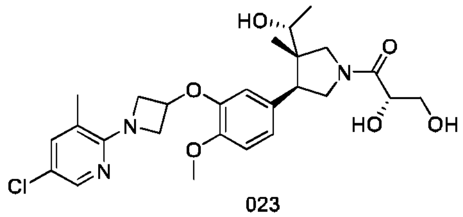

023

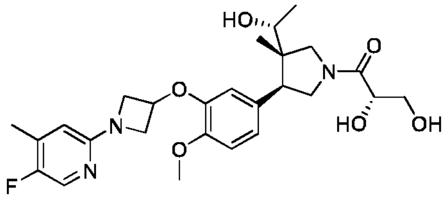

025

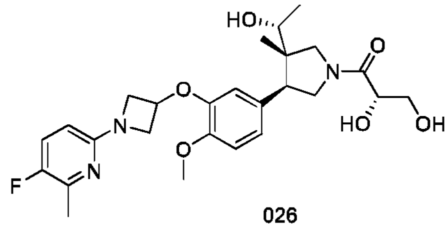

026

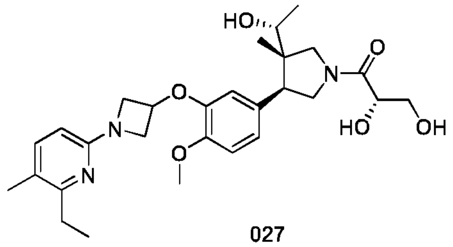

027

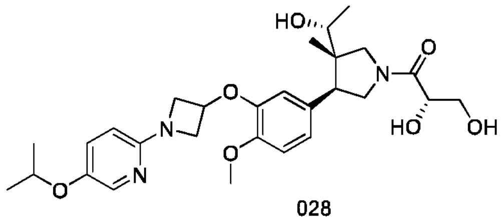

028

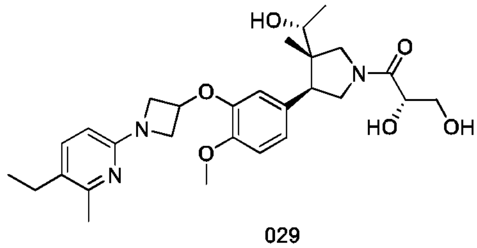

029

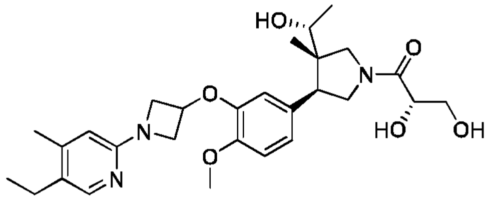

030

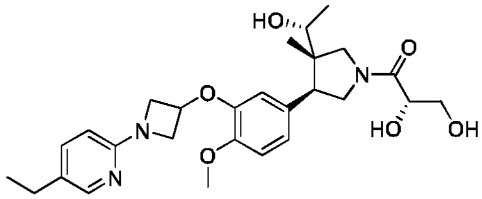

031

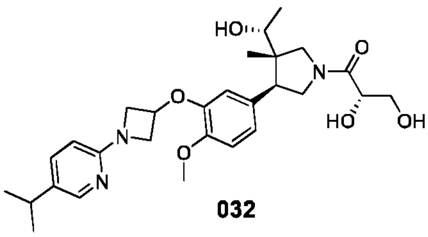

032

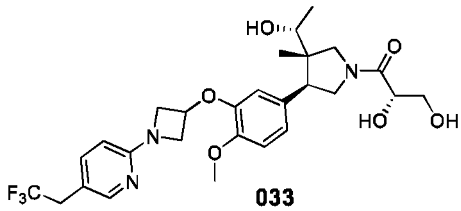

033

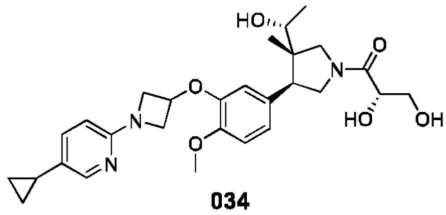

034

9. The compound according to claim 1, wherein the salt is a pharmaceutically acceptable salt, and comprises at least one of: sulfuric acid, phosphoric acid, nitric acid, hydrobromic acid, hydrochloric acid, formic acid, acetic acid, propionic acid, benzenesulfonic acid, benzoic acid, phenylacetic acid, salicylic acid, alginic acid, anthranilic acid, camphoric acid, citric acid, vinyl sulfonic acid, formic acid, fumaric acid, furoic acid, gluconic acid, glucuronic acid, glutamic acid, glycolic acid, isethionic acid, lactic acid, maleic acid, malic acid, mandelic acid, mucic acid, pamoic acid, pantothenic acid, stearic acid, succinic acid, sulfanilic acid, tartaric acid, p-toluenesulfonic acid, malonic acid, 2-hydroxypropionic acid, oxalic acid, glycolic acid, glucuronic acid, galacturonic acid, citric acid, lysine, arginine, aspartic acid, cinnamic acid, p-toluenesulfonic acid, methanesulfonic acid, ethanesulfonic acid, or trifluoromethanesulfonic acid.

10. A pharmaceutical composition, comprising:

the compound according to any one of claims 1 to 9 as an active ingredient; and
an additional and selectable drug used for treatment or prevention of at least one of: a PDE4-related disease, an inflammatory disease, an allergic disease, an autoimmune disease, transplant rejection, an arthritic disease, a skin inflammatory disease, an inflammatory bowel disease and a disease related to smooth muscle contractility, asthma, chronic bronchitis, chronic obstructive pneumonia, allergic rhinitis, respiratory distress syndrome, adult respiratory distress syndrome, idiopathic dermatitis, psoriasis, urticaria, rheumatoid arthritis, osteoarthritis, gouty arthritis or spondylitis, ulcerative colitis, Crohn's disease and overactive bladder, frequent urination, and urgent urination.

11. A use of the compound according to any one of claims 1 to 9 or the pharmaceutical composition according to claim 10 in a preparation of a drug for treatment or prevention of a PDE4-related disease.

12. A use of the compound according to any one of claims 1 to 9 or the pharmaceutical composition according to claim 10 in a preparation of a drug for treatment or prevention of at least one of: an inflammatory disease, an allergic disease, an autoimmune disease, transplant rejection, an arthritic disease, a skin inflammatory disease, an inflammatory bowel disease and a disease related to smooth muscle contractility, asthma, chronic bronchitis, chronic obstructive pneumonia, allergic rhinitis, respiratory distress syndrome, adult respiratory distress syndrome, idiopathic dermatitis, psoriasis, urticaria, rheumatoid arthritis, osteoarthritis, gouty arthritis or spondylitis, ulcerative colitis, Crohn's disease and overactive bladder, frequent urination, and urgent urination.

13. A drug combination, comprising:

the compound according to any one of claims 1 to 9 as an active ingredient; and
an additional drug used for treatment or prevention of at least one of: a PDE4-related disease, an inflammatory disease, an allergic disease, an autoimmune disease, transplant rejection, an arthritic disease, a skin inflammatory disease, an inflammatory bowel disease and a disease related to smooth muscle contractility, asthma, chronic bronchitis, chronic obstructive pneumonia, allergic rhinitis, respiratory distress syndrome, adult respiratory distress syndrome, idiopathic dermatitis, psoriasis, urticaria, rheumatoid arthritis, osteoarthritis, gouty

arthritis or spondylitis, ulcerative colitis, Crohn’s disease and overactive bladder, frequent urination, and urgent urination.

**14.** A method for treating or preventing a PDE4-related disease, comprising:
administrating the compound according to any one of claims 1 to 9 or the pharmaceutical composition according to claim 10 or the drug combination according to claim 13 to a patient.

**15.** The method according to claim 14, wherein the PDE4-related disease comprises at least one of: an inflammatory disease, an allergic disease, an autoimmune disease, transplant rejection, an arthritic disease, a skin inflammatory disease, an inflammatory bowel disease and a disease related to smooth muscle contractility, asthma, chronic bronchitis, chronic obstructive pneumonia, allergic rhinitis, respiratory distress syndrome, adult respiratory distress syndrome, idiopathic dermatitis, psoriasis, urticaria, rheumatoid arthritis, osteoarthritis, gouty arthritis or spondylitis, ulcerative colitis, Crohn’s disease and overactive bladder, frequent urination, and urgent urination.

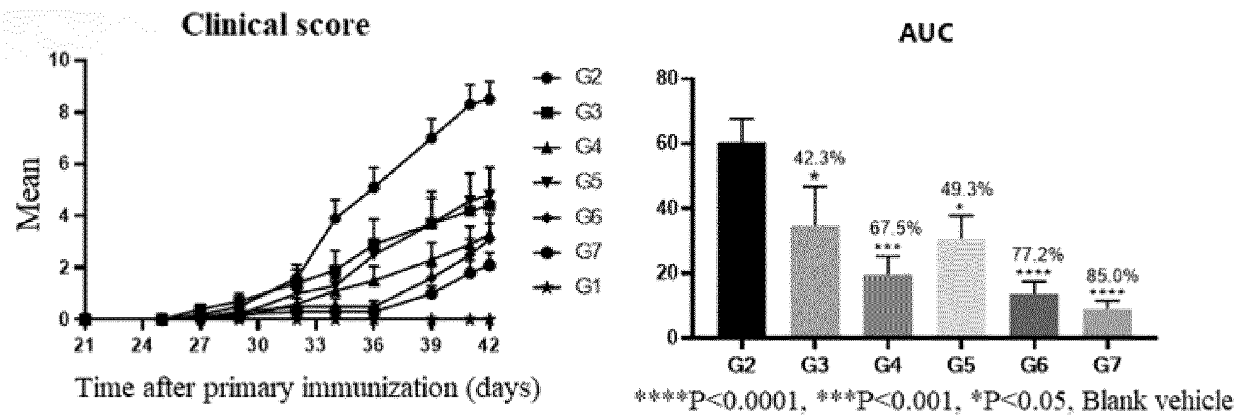
Clinical score
Mean
Time after primary immunization (days)
G2
G3
G4
G5
G6
G7
G1
AUC
42.3%
67.5%
49.3%
77.2%
85.0%
****P<0.0001, ***P<0.001, *P<0.05, Blank vehicle

FIG. 1

**INTERNATIONAL SEARCH REPORT**

International application No.
**PCT/CN2019/125983**

**A. CLASSIFICATION OF SUBJECT MATTER**

C07D 401/14(2006.01)i; C07D 403/14(2006.01)i; A61K 31/4439(2006.01)i; A61K 31/496(2006.01)i; A61K 31/497(2006.01)i; A61K 31/506(2006.01)i; A61P 13/10(2006.01)i; A61P 1/04(2006.01)i; A61P 11/00(2006.01)i; A61P 13/00(2006.01)i; A61P 17/00(2006.01)i; A61P 19/00(2006.01)i; A61P 37/00(2006.01)i

According to International Patent Classification (IPC) or to both national classification and IPC

**B. FIELDS SEARCHED**

Minimum documentation searched (classification system followed by classification symbols)

C07D; A61K 31/-; A61P

Documentation searched other than minimum documentation to the extent that such documents are included in the fields searched

Electronic data base consulted during the international search (name of data base and, where practicable, search terms used)

CNABS, CNKI, VEN, CAplus, Registry: 氮杂环丁烷, 吡咯烷, 磷酸二酯酶, PDE4, 抑制, azetidin, pyrrolidin, PHOSPHODIESTERASE, inhibit, structure search

**C. DOCUMENTS CONSIDERED TO BE RELEVANT**

| Category* | Citation of document, with indication, where appropriate, of the relevant passages | Relevant to claim No. |
|---|---|---|
| X | CN 106795137 A (ELI LILLY AND COMPANY) 31 May 2017 (2017-05-31) see claims 1, 2, and 7-13, description, paragraphs 5, 6, 27, and 36-42, and mebodiments 1-13 | 1-13 |
| A | CN 1434813 A (ICOS CORPORATION) 06 August 2003 (2003-08-06) see entire document | 1-13 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 March 2020** | **20 March 2020** |

| Name and mailing address of the ISA/CN | Authorized officer |
|---|---|
| **China National Intellectual Property Administration (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2019/125983**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **14-15**
because they relate to subject matter not required to be searched by this Authority, namely:

   [1] The subject matter of claims 14 and 15 relates to a method for the treatment of the human or an animal body, which falls within the cases set out in PCT Rule 39.1(iv) for which an international search is not required.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2019/125983**

| Patent document cited in search report | | | Publication date (day/month/year) | Patent family member(s) | | | Publication date (day/month/year) |
|---|---|---|---|---|---|---|---|
| CN | 106795137 | A | 31 May 2017 | JP | 2017527587 | A | 21 September 2017 |
| | | | | EA | 030034 | B1 | 29 June 2018 |
| | | | | EA | 201790156 | A1 | 30 June 2017 |
| | | | | KR | 20170040803 | A | 13 April 2017 |
| | | | | TW | 201625591 | A | 16 July 2016 |
| | | | | DK | 3191466 | T3 | 20 January 2020 |
| | | | | JP | 6285610 | B2 | 28 February 2018 |
| | | | | WO | 2016040083 | A1 | 17 March 2016 |
| | | | | PE | 28 March 2017 | A1 | 19 April 2017 |
| | | | | PE | 03282017 | A1 | 19 April 2017 |
| | | | | EP | 3191466 | A1 | 19 July 2017 |
| | | | | KR | 101857931 | B1 | 14 May 2018 |
| | | | | US | 2017240532 | A1 | 24 August 2017 |
| | | | | ZA | 201700555 | B | 19 December 2018 |
| | | | | AU | 2015315533 | A1 | 23 February 2017 |
| | | | | BR | 112017002852 | A2 | 26 December 2017 |
| | | | | AP | 201709778 | A0 | 31 March 2017 |
| | | | | CA | 2955634 | A1 | 17 March 2016 |
| | | | | US | 2020002318 | A1 | 02 January 2020 |
| | | | | MX | 2017003138 | A | 23 May 2017 |
| | | | | TN | 2017000047 | A1 | 04 July 2018 |
| | | | | CL | 2017000484 | A1 | 10 November 2017 |
| | | | | PH | 12017500459 | A1 | 31 July 2017 |
| | | | | AU | 2015315533 | B2 | 15 March 2018 |
| | | | | NZ | 729087 | A | 27 July 2018 |
| | | | | SG | 11201701225 Y | A | 30 March 2017 |
| | | | | AP | 201709778 | D0 | 31 March 2017 |
| | | | | EP | 3191466 | B1 | 04 December 2019 |
| | | | | AR | 101696 | A1 | 04 January 2017 |
| | | | | CR | 20170050 | A | 25 April 2017 |
| CN | 1434813 | A | 06 August 2003 | JP | 2003519135 | A | 17 June 2003 |
| | | | | EA | 200200590 | A1 | 26 December 2002 |
| | | | | HR | P20020541 | B1 | 30 June 2008 |
| | | | | SK | 9172002 | A3 | 06 November 2002 |
| | | | | EP | 1242400 | B1 | 03 May 2006 |
| | | | | US | 6998416 | B2 | 14 February 2006 |
| | | | | UA | 73964 | C2 | 17 October 2005 |
| | | | | EE | 200200351 | A | 15 October 2003 |
| | | | | BR | 0016693 | A | 11 March 2003 |
| | | | | US | 6458787 | B1 | 01 October 2002 |
| | | | | AT | 325108 | T | 15 June 2006 |
| | | | | US | 6258833 | B1 | 10 July 2001 |
| | | | | PL | 355988 | A1 | 31 May 2004 |
| | | | | RS | 50841 | B | 31 August 2010 |
| | | | | BR | PI0016693 | B1 | 01 September 2015 |
| | | | | TR | 200400702 | T4 | 21 June 2004 |
| | | | | IL | 150313 | D0 | 01 December 2002 |
| | | | | US | 2004152754 | A1 | 05 August 2004 |
| | | | | AU | 778543 | B2 | 09 December 2004 |
| | | | | BG | 65892 | B1 | 30 April 2010 |

Form PCT/ISA/210 (patent family annex) (January 2015)

**INTERNATIONAL SEARCH REPORT**
**Information on patent family members**

International application No.
**PCT/CN2019/125983**

| Patent document cited in search report | Publication date (day/month/year) | Patent family member(s) | | | Publication date (day/month/year) |
|---|---|---|---|---|---|
| | | CA | 2395193 | A1 | 05 July 2001 |
| | | HU | 0301113 | A3 | 28 July 2011 |
| | | ZA | 200205551 | A | 11 July 2003 |
| | | HU | 0301113 | A2 | 28 August 2003 |
| | | ZA | 200205551 | B | 11 July 2003 |
| | | BG | 106860 | A | 31 March 2003 |
| | | EA | 005686 | B1 | 28 April 2005 |
| | | NO | 20023008 | D0 | 21 June 2002 |
| | | MX | PA02006212 | A | 26 February 2004 |
| | | EP | 1242400 | A1 | 25 September 2002 |
| | | DE | 60027769 | D1 | 08 June 2006 |
| | | JP | 4294903 | B2 | 15 July 2009 |
| | | YU | 48002 | A | 28 November 2005 |
| | | US | 2004023945 | A1 | 05 February 2004 |
| | | HR | P20020541 | A2 | 31 August 2004 |
| | | AU | 2426201 | A | 09 July 2001 |
| | | US | 6423710 | B1 | 23 July 2002 |
| | | CZ | 20022188 | A3 | 12 March 2003 |
| | | EE | 05087 | B1 | 15 October 2008 |
| | | CA | 2395193 | C | 17 June 2008 |
| | | NO | 20023008 | L | 20 August 2002 |
| | | IS | 6436 | A | 19 June 2002 |
| | | US | 7176229 | B2 | 13 February 2007 |
| | | SK | 287964 | B6 | 03 July 2012 |
| | | US | 6716871 | B2 | 06 April 2004 |
| | | WO | 0147905 | A1 | 05 July 2001 |
| | | GE | P20043384 | B | 10 May 2004 |
| | | PL | 202227 | B1 | 30 June 2009 |
| | | NO | 20023008 | A | 20 August 2002 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 201910806179
- CN 201811553504
- WO 2016040083 A1
- WO 2001047905 A1
- CN 106795137 A
- WO 2014159012 A1


### Non-patent literature cited in the description


- **NICHOLS, P. J. ; DEMATTEI, J. A.** *Org. Lett.,* 2006, vol. 8, 1495-1498